# EUROPEAN PATENT APPLICATION

(11) **EP 3 819 371 A1**
(43) Date of publication of application: **12.05.2021**
(21) Application number: 19830561.7
(22) Date of filing: 05.07.2019
(51) Int. Cl.: C12N 1/20, C02F 3/34

(54) **NOVEL MICROORGANISMS FOR DECOMPOSING OIL AND FAT CONTAINING FATTY ACID**

(30) Priority: 06.07.2018 JP 2018129506
(71) Applicant: National University Corporation Tokai National Higher Education and Research System, Nagoya-shi Aichi 464-8601 (JP)
(72) Inventor: HORI, Katsutoshi, Nagoya-shi, Aichi 464-8601 (JP)
(74) Representative: Engelhard, Markus
(86) International application number: PCT/JP2019/026850
(87) International publication number: WO 2020/009232

(57) **Abstract**

The present disclosure provides a new microorganism with an ability to decompose oil and fat. In one embodiment, the microorganism is from the family Burkholderiaceae. In one embodiment, the microorganism is from the genus Burkholderia. In one embodiment, the microorganism is Burkholderia arboris, or Burkholderia cepacia complex, or the like. In one embodiment, the microorganism of the present disclosure has an ability to assimilate trans fatty acid. In one embodiment, the microorganism of the present disclosure has an ability to decompose trans fatty acid. In one embodiment, the microorganism of the present disclosure has an ability to decompose trans fatty acid-containing oil and fat. In one embodiment, the microorganism of the present disclosure has an ability to assimilate trans fatty acid-containing oil and fat.

## Description

### [Technical Field]

The present disclosure relates to a microorganism having an ability to decompose oil and fat and/or fatty acid and use thereof. More specifically, the present disclosure relates to a novel microorganism that decomposes a trans fatty acid-containing oil and fat.

### [Background Art]

Wastewater from a food factory or oil and fat factory has high oil content. Such oil content causes deterioration in various biological treatment functions, such as a reduced capacity for treatment with activated sludge, imperfect separation of solids from liquids due to reduced precipitation, fouling of a membrane in membrane bioreactor (MBR), and suppression of methane fermentation in anaerobic digestion. For this reason, oil content is removed with, for example, a dissolved air flotation apparatus or the like as a preliminary step of biological treatment of wastewater with high oil content. Since kitchen wastewater produced by the restaurant industry also has high oil content, a grease trap for removing oil content is installed. Both dissolved air flotation apparatuses and grease traps face problems such as the apparatuses being a source of foul odor or pests, the cost associated with the collection/transport of the separated oil and industrial waste processing, labor and cost associated with maintenance, cleaning, and the like. While oil decomposing technologies using microorganisms have been considered as means for solving such problems and multiple related microbial formulations are commercially available, it is extremely difficult to reduce the concentration of oil content by microorganism decomposition to a desired level within a configurable retention time. For this reason, a dissolved air flotation apparatus or a conventional grease trap is currently used in most cases.

When oil content is high, fermentation of food waste also faces problems such as suppression of fermentation or high content of oil in wastewater in an extinguishing type of disposer. Further, oily sludge that is separated and collected with a dissolved air flotation apparatus or a grease trap is industrial waste, so treatment thereof is very costly. In this regard, decomposition of such oil content with microorganisms is being considered, but the reality is that the ability to decompose of microorganisms is also limited in the same manner as the aforementioned wastewater treatment.

The decomposition rate is an issue in the removal of oil content using microorganisms as described above. In particular, reduced activity due to low temperatures during winter often makes it challenging to apply microorganisms. Especially at low temperature during the winter, the decomposition rate of oil and fat by microorganisms is extremely low, such that wastewater treatment or waste treatment using specific microorganisms is considered impossible.

### [Citation List]

### [Non Patent Literature]

[NPL 1] Journal of Bioscience and Biotechnology, Vol. 107, No. 4 401-408, 2009

### [Summary of Invention]

### [Solution to Problem]

As a result of diligent studies, the inventors found a novel microorganism in the family Burkholderiaceae that decomposes trans fatty acid-containing oil and fat. In some aspects, this microorganism was found to be capable of efficiently decomposing oil and fat and/or fatty acid even at a low temperature of about 15°C. The present disclosure also relates to application of the microorganism of the present disclosure such as for oil treatment.

The present disclosure provides a new microorganism having an ability to decompose oil and fat and a method of decomposing oil and fat and/or fatty acid using such a microorganism.

Therefore, the present disclosure provides the following.
(Item 1) A bacterium in the family Burkholderiaceae, having an ability to assimilate trans fatty acid-containing oil and fat.
(Item 2) A bacterium in the family Burkholderiaceae, having an ability to assimilate trans fatty acid.
(Item 3) A bacterium in the family Burkholderiaceae, having an ability to decompose trans fatty acid.
(Item 4) A bacterium in the family Burkholderiaceae, having an ability to decompose trans fatty acid-containing oil and fat.
(Item 5) A bacterium in the family Burkholderiaceae, having an ability to decompose oil and fat at 15°C.
(Item 6) The bacterium in the family Burkholderiaceae of any one of items 1 to 4, wherein the ability to assimilate or decompose is retained at 15°C.
(Item 7) The bacterium of any one of items 1 to 6, which is a bacterium of the genus Burkholderia.
(Item 8) The bacterium of any one of items 1 to 7, which is a microorganism belonging to Burkholderia arboris, Burkholderia ambifaria, or Burkholderia cepacia complex.
(Item 9) The bacterium of any one of items 1 to 8, which is a KH-1 strain (bacterial strain identified by accession number NITE BP-02731), a KH-1AL1 strain (bacterial strain identified by receipt number NITE ABP-02977), a KH-1AL2 strain (bacterial strain identified by receipt number NITE ABP-02978), or a KH-1AL3 strain (bacterial strain identified by receipt number NITE ABP-02979) of a bacterium in the genus Burkholderia, or a derivative strain thereof that has a feature of the bacterium of any one or more of items 1 to 7.
(Item 10) An oil decomposing agent comprising the bacterium of any one of items 1 to 9.
(Item 11) The oil decomposing agent of item 10, further comprising an oil treating component.
(Item 12) A kit for decomposing oil, comprising the bacterium of any one of items 1 to 9 or the oil decomposing agent of item 10, and an additional oil treating component.
(Item 13) An method of decomposing and removing oil, comprising causing the bacterium of any one of items 1 to 9 or the oil decomposing agent of item 10 or 11 to act on a subject of treatment.
(Item 14) The method of item 13, wherein the subject of treatment comprises trans fatty acid or trans fatty acid-containing oil and fat.

The present disclosure is intended so that one or more of the features described above can be provided not only as the explicitly disclosed combinations, but also as other combinations. Additional embodiments and advantages of the present disclosure are recognized by those skilled in the art by reading and understanding the following detailed description as needed.

### [Advantageous Effects of Invention]

The microorganism of the present disclosure and a composition comprising the same are capable of decomposing trans fatty acid and trans fatty acid-containing oil and fat, and thus are capable of treating an oil and fat-containing subject, particularly wastewater discharged from a food factory or the like, and may also be capable of handling a broad range of oil and fat concentrations and may be usable at a lower temperature than conventional art. Therefore, the microorganism and composition can be applied in a broad range of situations, such as cleanup of an environmental contamination by oil and fat, food waste treatment, composting treatment, waste treatment such as wastewater treatment, and composting.

The present disclosure can also solve the problem of oil and fat which is difficult to decompose, i.e., oil type. The microorganism of the present disclosure and a composition comprising the same can decompose trans fatty acid and oil and fat containing the same generated in the hydrogenation process of oil and fat, and attains an effect of being able to treat especially margarine, fat spread, shortening, or the like containing a large amount of such fatty acid-containing oil and fat, which could not be treated with conventional microorganisms. In particular, the present disclosure provides an effect of achieving decomposition of trans fatty acid, which is available at a practical level as bacteria that can be the primary microorganisms for treating wastewater or waste. The microorganism of the present disclosure and a composition comprising the same can also enable decomposition of oil and fat or fatty acid at a low temperature.

### [Brief Description of Drawings]

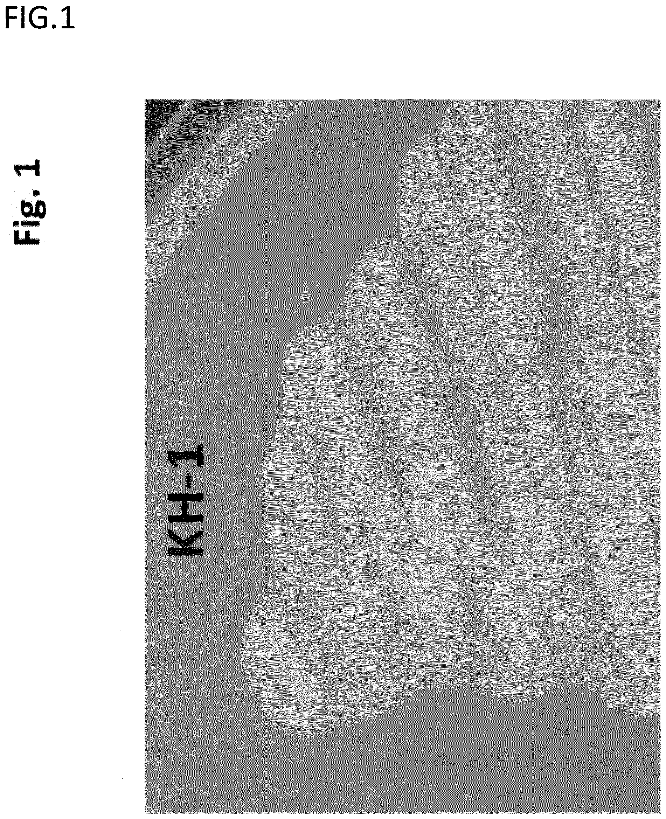

[Figure 1] Figure **1** shows a result of culturing Burkholderia arboris KH-1 strain for 5 days at 15°C on a canola oil-supplemented medium.

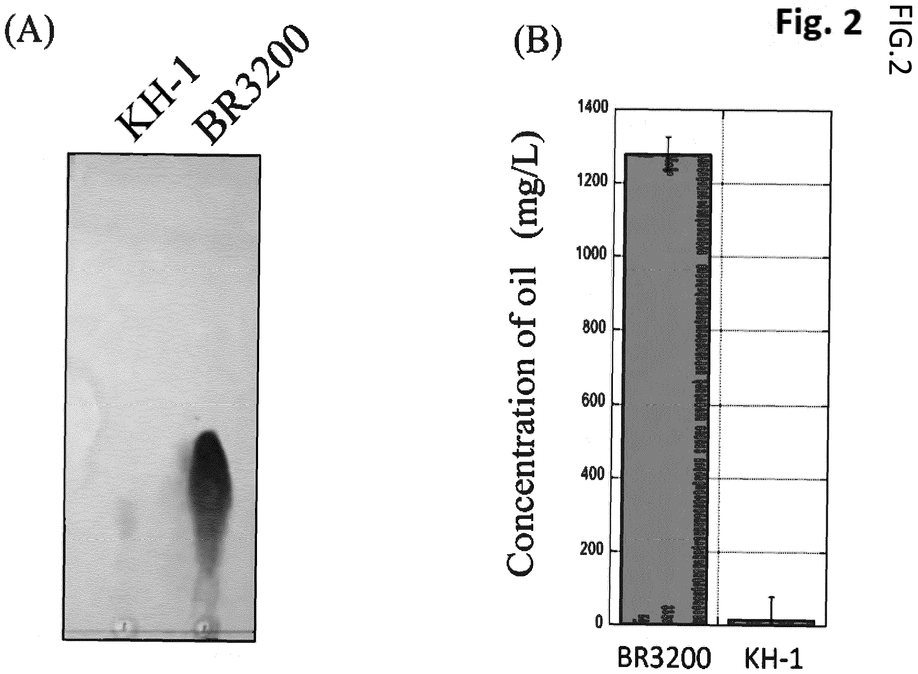

[Figure 2] Figure **2** shows a comparison of elaidic acid decomposition activities of KH-1 and BioRemove 3200 (BR3200) (Novozymes, Denmark). The bacteria were cultured for 24 hours at 28°C and at 130 rpm. (A) is a picture of TLC analysis showing fatty acid in culture. The left column shows the result from KH-1, and the right column shows the result from BR3200. (B) is a result of measuring oil content corresponding to a normal hexane value with an oil content measuring reagent kit. The left side shows the result from BR3200, and the right side shows the result from KH-1. The error bar indicates the standard deviation.

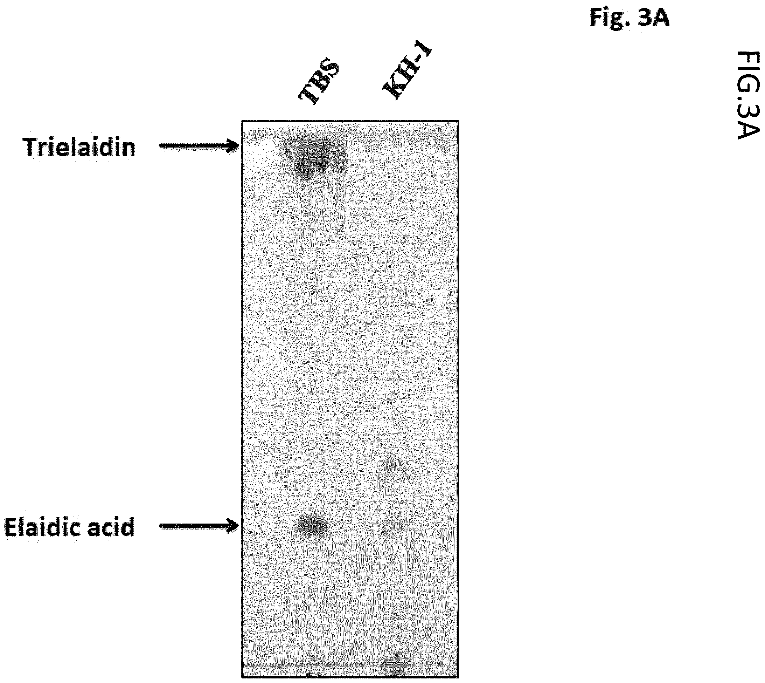

[Figure 3A] Figure **3A** shows the elaidic acid-containing oil and fat (trielaidin) decomposition activity of KH-1. The bacteria were cultured for 5 days at 28°C and at 130 rpm. This is a picture of TLC analysis showing glyceride and fatty acid in the culture. The left column is a result from TBS buffer, and the right column is the result from KH-1.

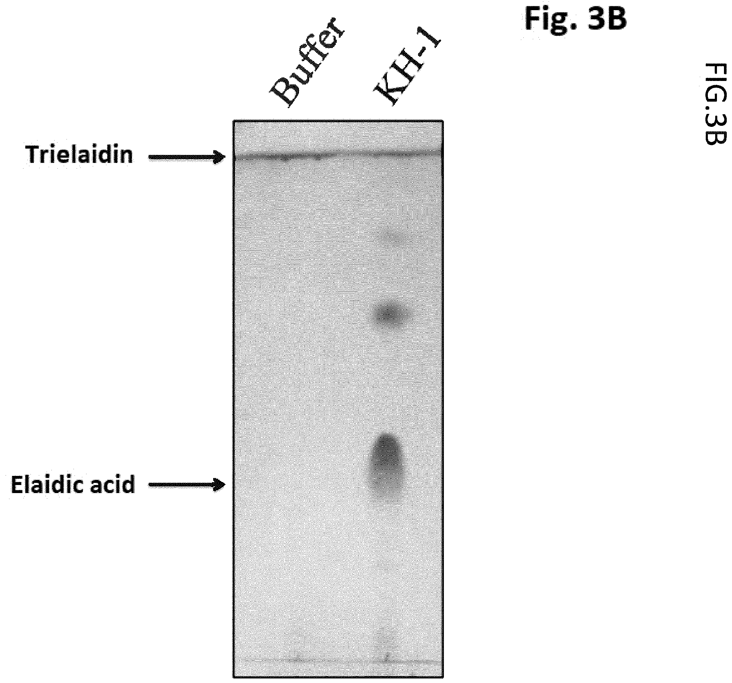

[Figure 3B] Figure **3B** shows the elaidic acid-containing oil and fat (trielaidin) decomposition activity of culture supernatant of KH-1. The bacteria were incubated for 24 hours at 28°C and at 130 rpm. This is a picture of TLC analysis showing glyceride and fatty acid in the solution. The left column is a result from TBS buffer, and the right column is the result from KH-1.

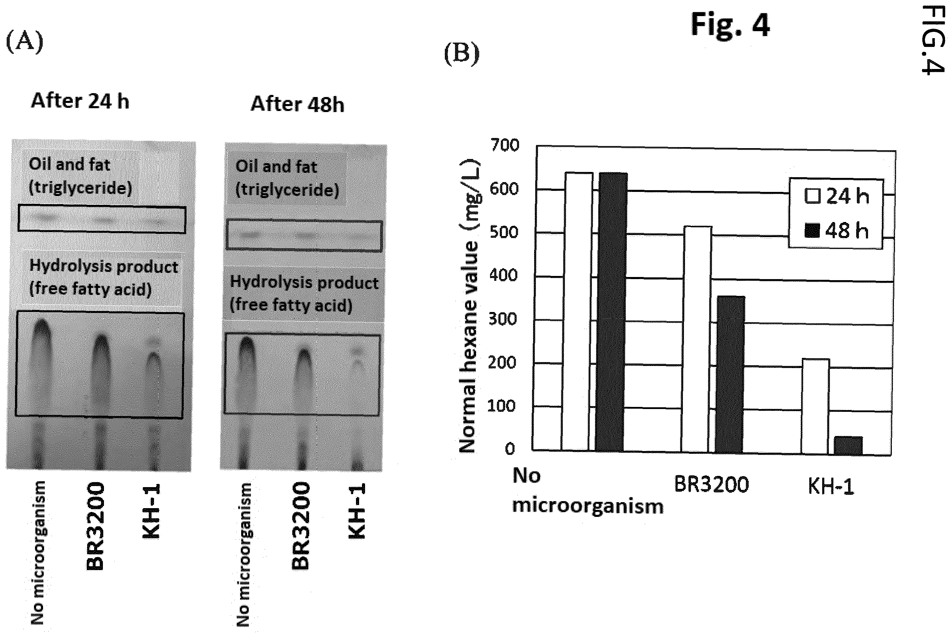

[Figure 4] Figure **4** shows a comparison of the abilities to decompose oil and fat of KH-1 and BioRemove 3200 (BR3200) using actual wastewater. The bacteria were cultured at 28°C and the samples were collected after 24 hours and 48 hours. (A) is a picture of TLC analysis showing residual oil content in wastewater (left side shows the results for 24 hours after starting the culture, and right side shows the results for 48 hours after starting the culture). Each column represents, from the left, result for no addition of microorganisms, result for BR3200 addition, and result for KH-1 addition. (B) is the result of measuring oil content corresponding to a normal hexane value with an oil content measuring reagent kit. Each column represents, from the left, result for no addition of microorganisms, result for BR3200 addition, and result for KH-1 addition.

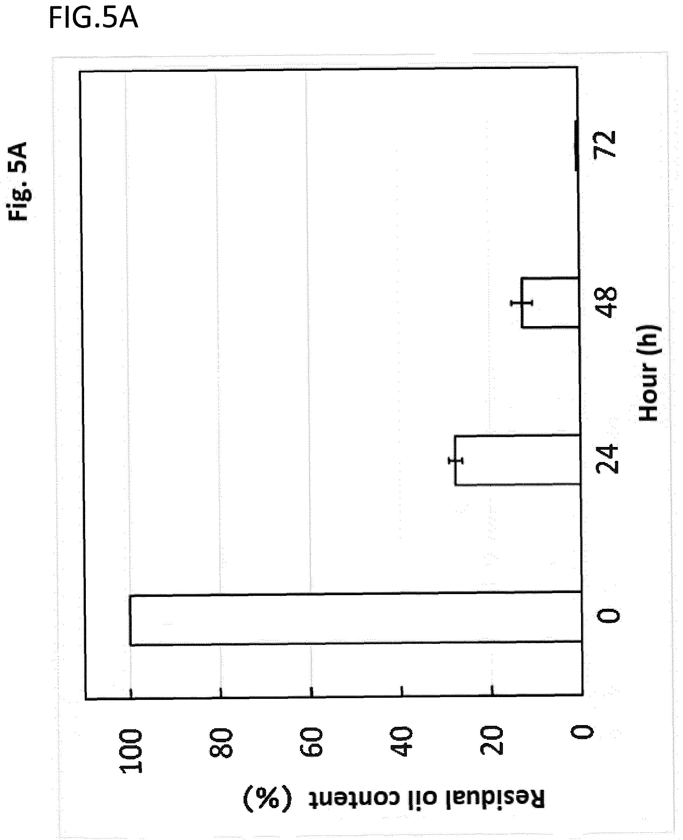

[Figure 5A] Figure **5A** shows decomposition of canola oil by KH-1 in 15°C culture (5L volume fermenter, 250 rpm, under 200 ml/min air circulation, and pH of 7.0). This is the result of measuring oil content corresponding to a normal hexane value with an oil content measuring reagent kit. The figure shows, from the left, results for 0 hours, 24 hours, 48 hours, and 72 hours after starting the culture. The vertical axis indicates the ratio when the measurement value at 0 hours is considered as 100% with the standard deviation.

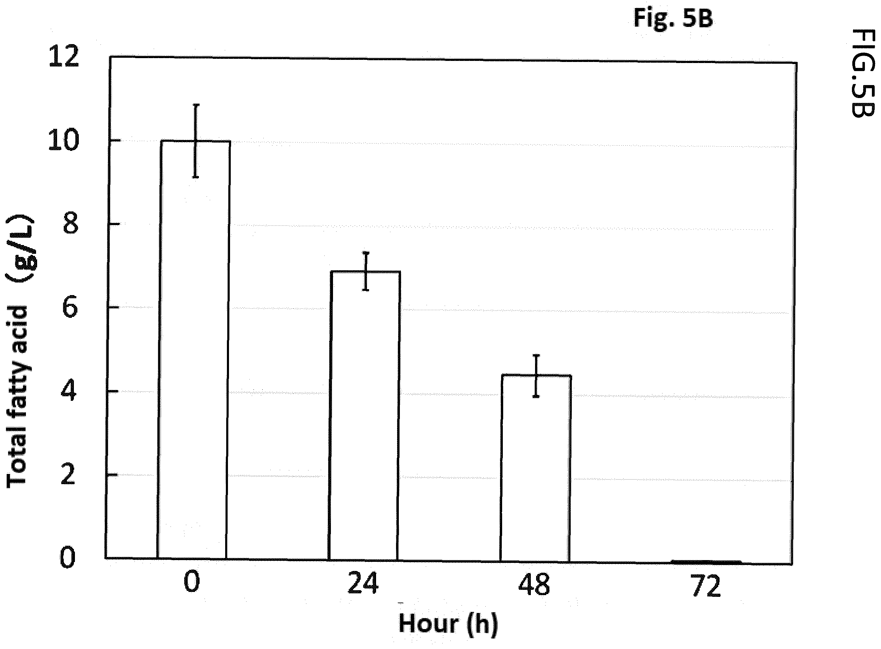

[Figure 5B] Figure **5B** shows decomposition of canola oil by KH-1 in 15°C culture (5L volume fermenter, 250 rpm, under 200 ml/min air circulation, and pH of 7.0). This is the result of quantifying the total fatty acid (total of fatty acid in triglyceride and free fatty acid) at 0 hours, 24, hours, 48 hours, and 72 hours after starting the culture by gas chromatography. The error bar indicates the standard deviation.

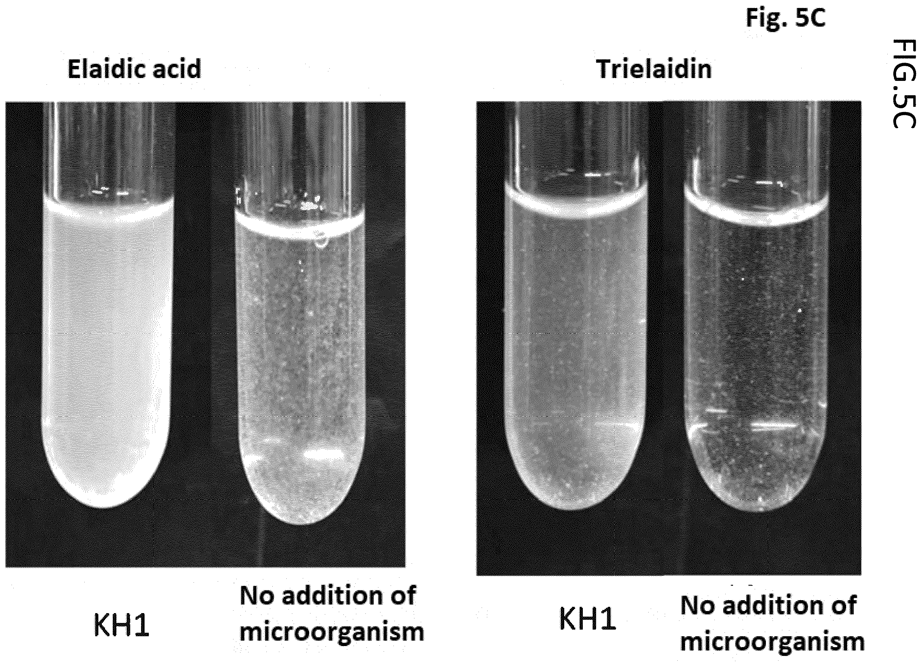

[Figure 5C] Figure **5C** shows the ability to assimilate elaidic acid or trielaidin of a KH-1 strain in 15°C culture. These are results of culturing for 5 days at 15°C in a medium having elaidic acid or trielaidin as the sole carbon source. The pictures are for, from the left, KH-1 added elaidic acid medium, elaidic acid medium with no addition of microorganisms, KH-1 added trielaidin medium, and trielaidin medium with no addition of microorganisms.

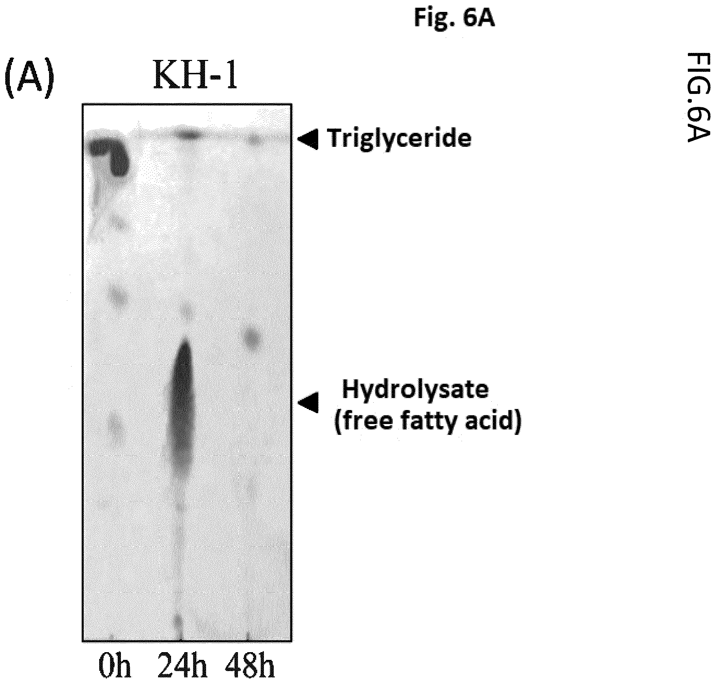

[Figure 6A] Figure **6A** shows the ability to decompose oil and fat (canola oil) of KH-1 in 28°C culture. This is a picture of thin-layer chromatography (TLC) analysis showing residual oil content in the culture. Each column shows, from the left, a result as of start of culture, and results at 24 hours and 48 hours after starting the culture.

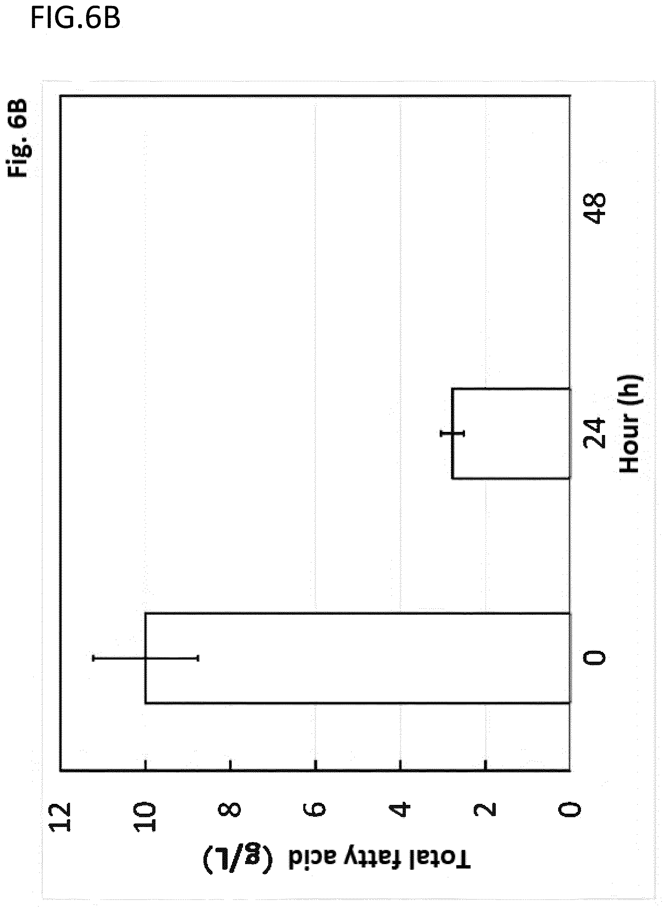

[Figure 6B] Figure **6B** shows the ability to decompose oil and fat of KH-1 in 28°C culture. This is the result of quantifying total fatty acid (total of fatty acid in triglyceride and free fatty acid) at 0 hours, 24 hours, and 48 hours after starting the culture by gas chromatography. The error bar indicates the standard deviation.

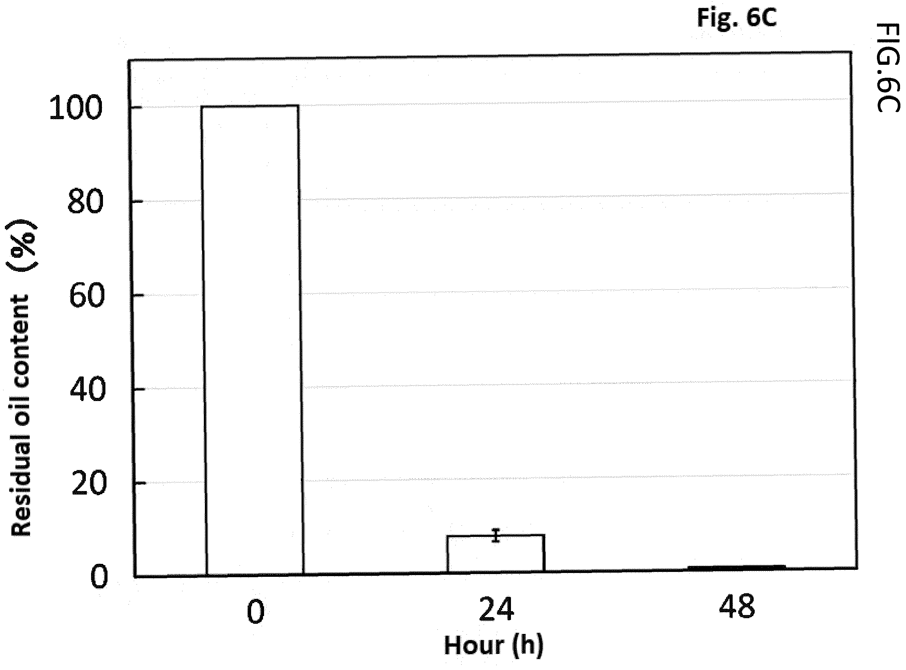

[Figure 6C] Figure **6C** shows the ability to decompose oil and fat of KH-1 in 28°C culture. This is the result of measuring oil content corresponding to a normal hexane value with an oil content measuring reagent kit. The figure shows, from the left, results for 0 hours, 24 hours, and 48 hours after starting the culture. The vertical axis indicates the ratio when the measurement value at 0 hours is considered as 100% with the standard deviation.

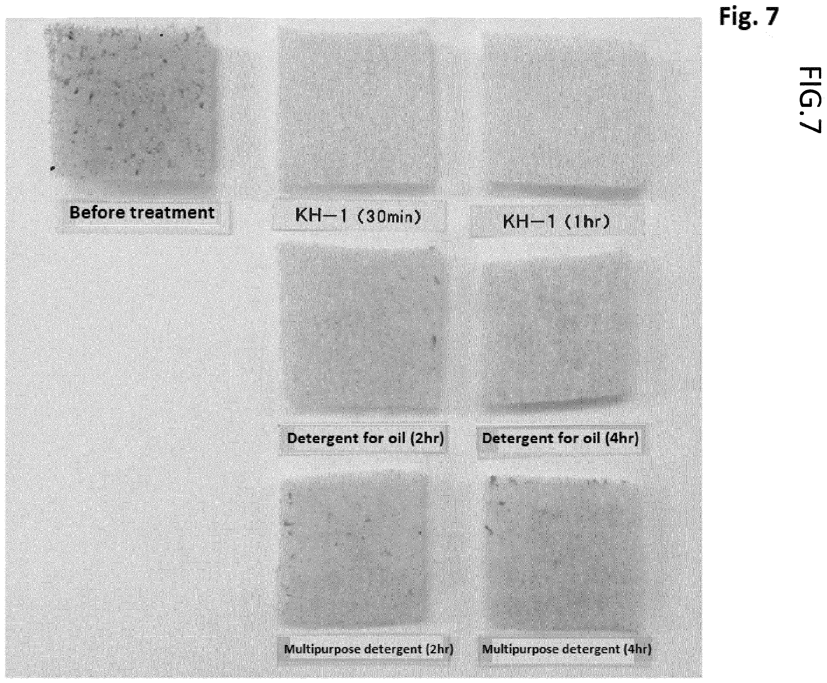

[Figure 7] Figure **7** shows a comparison of KH-1 with detergents. The pictures show ventilator filters with oil stain that were washed by leaving the filters soaked in a culture supernatant of KH-1, a detergent for oil, and a multipurpose detergent. The left column shows the state prior to treatment. The center and right columns show top row: KH-1 treatment (center column: 30 minutes, right column: 1 hour), middle row: treatment with a detergent for oil (center column: 2 hours, right column: 4 hours), and bottom row: treatment with a multipurpose detergent (center column: 2 hours, right column: 4 hours).

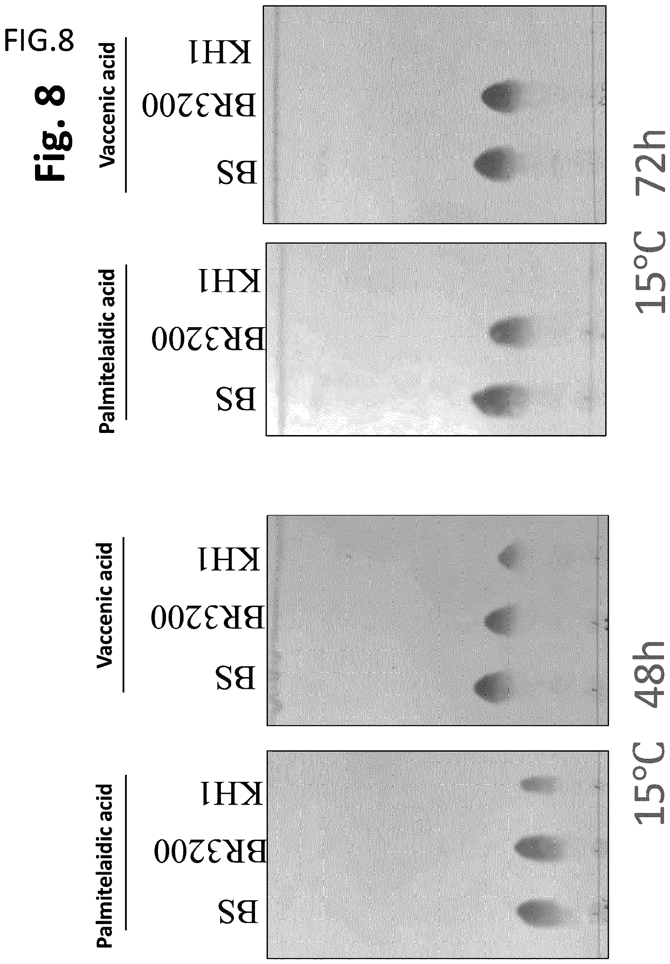

[Figure 8] Figure **8** shows a comparison of palmitelaidic acid and vaccenic acid decomposition activities of KH-1 and BioRemove 3200 (BR3200) (Novozymes, Denmark). The figure shows pictures of TLC analysis showing fatty acid in culture after 48 hours or 72 hours of culture at 15°C. The panels are, from the left, 48 hour culture with palmitelaidic acid, 48 hour culture with vaccenic acid, 72 hour culture with palmitelaidic acid, and 72 hour culture with vaccenic acid. In each panel, the left side is for the negative control (BS), the center is for BR3200, and right side is for KH-1.

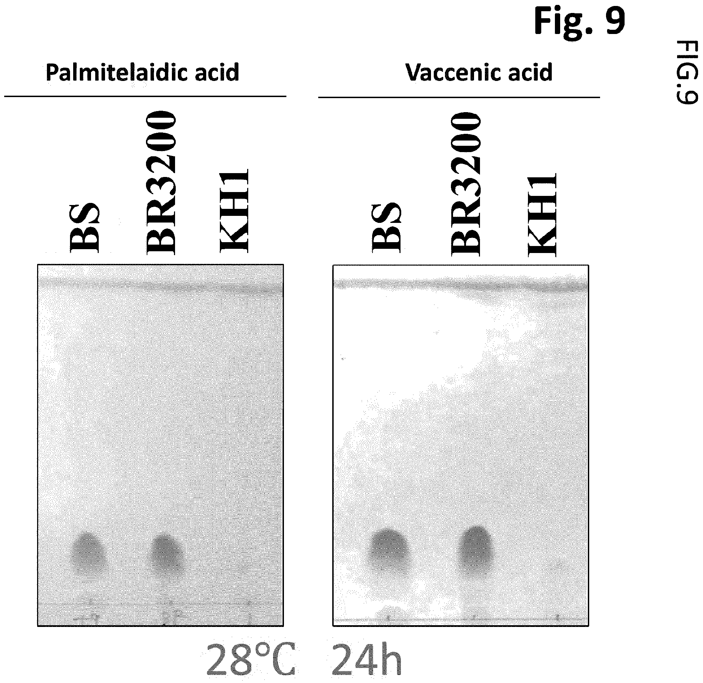

[Figure 9] Figure **9** shows a comparison of palmitelaidic acid and vaccenic acid decomposition activities of KH-1 and BioRemove 3200 (BR3200) (Novozymes, Denmark). The figure shows pictures of TLC analysis showing fatty acid in the culture after 24 hours of culture at 28°C. The left panel is for culture with palmitelaidic acid, and the right panel is for culture with vaccenic acid. In each panel, the left side is for the negative control (BS), the center is for BR3200, and right side is for KH-1.

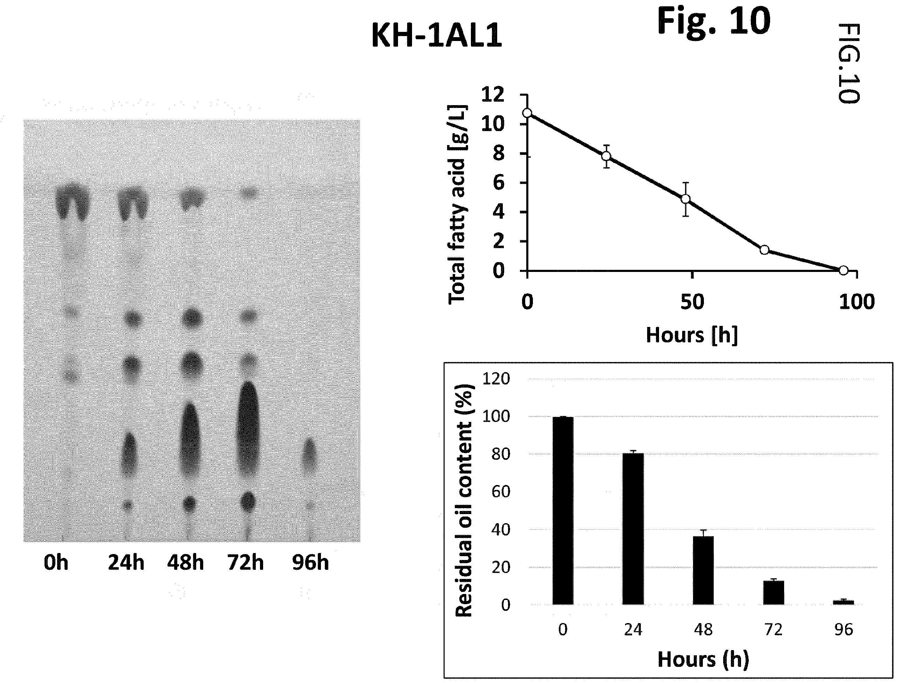

[Figure 10] Figure **10** shows decomposition of canola oil by KH-1AL1 in 15°C culture. The figure shows results at 0 hours, 24 hours, 48 hours, 72 hours, and 96 hours after starting the culture. The left side is a picture of TLC analysis showing residual oil content in the culture. The top right is the result of quantifying total fatty acid (total of fatty acid in triglyceride and free fatty acid) by gas chromatography. The vertical axis indicates the total fatty acid concentration (error bar indicates the standard deviation). The bottom right is a result of measuring oil content corresponding to a normal hexane value with an oil content measuring reagent kit. The vertical axis indicates the ratio of residual oil content when the measurement value at 0 hours is considered as 100% (error bar indicates the standard deviation).

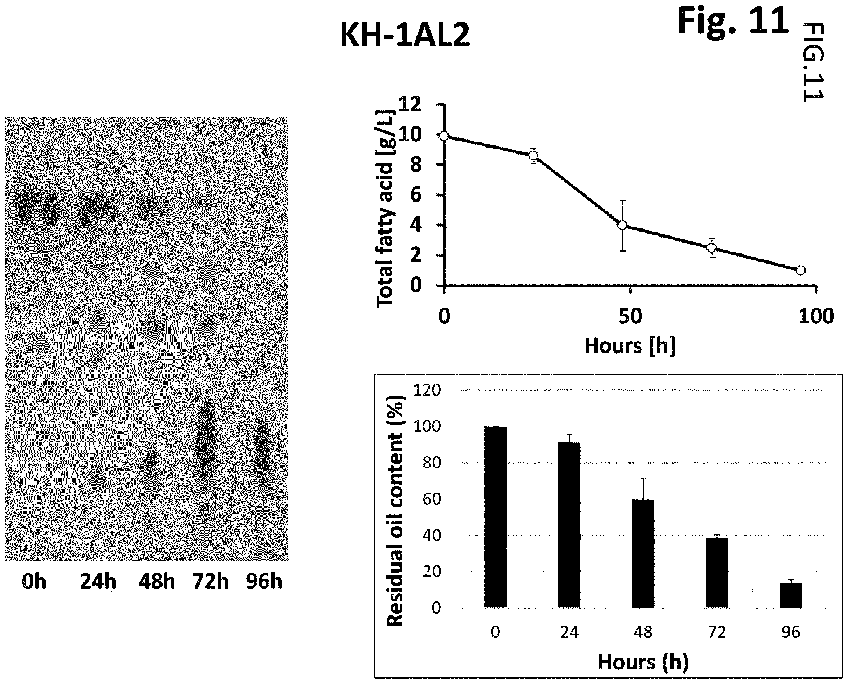

[Figure 11] Figure **11** shows decomposition of canola oil by KH-1AL2 in 15°C culture. The figure shows results at 0 hours, 24 hours, 48 hours, 72 hours, and 96 hours after starting the culture. The left side is a picture of TLC analysis showing residual oil content in the culture. The top right is the result of quantifying total fatty acid (total of fatty acid in triglyceride and free fatty acid) by gas chromatography. The vertical axis indicates the total fatty acid concentration (error bar indicates the standard deviation). The bottom right is a result of measuring oil content corresponding to a normal hexane value with an oil content measuring reagent kit. The vertical axis indicates the ratio of residual oil content when the measurement value at 0 hours is considered as 100% (error bar indicates the standard deviation).

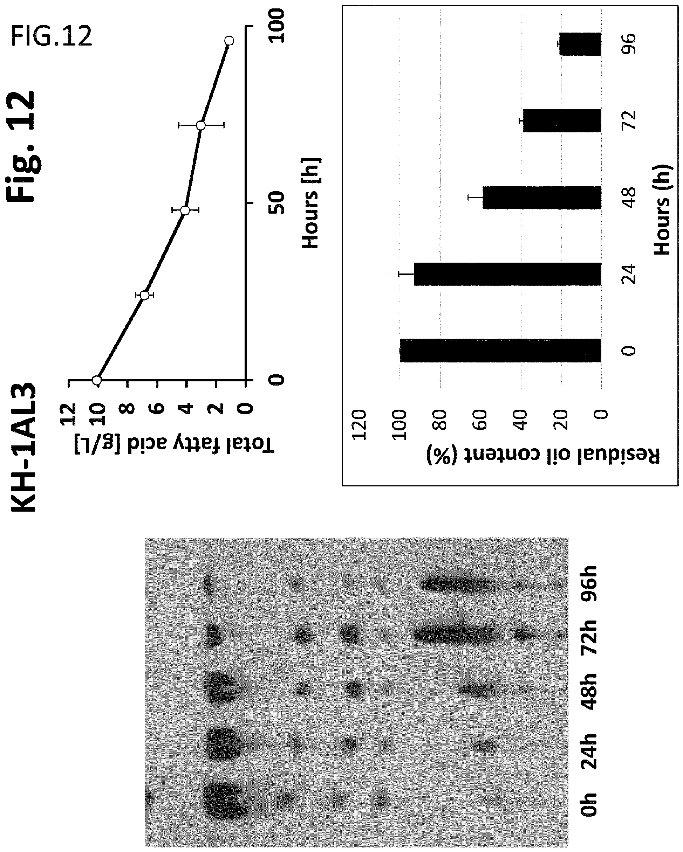

[Figure 12] Figure **12** shows decomposition of canola oil by KH-1AL3 in 15°C culture. The figure shows results at 0 hours, 24 hours, 48 hours, 72 hours, and 96 hours after starting the culture. The left side is a picture of TLC analysis showing residual oil content in the culture. The top right is the result of quantifying total fatty acid (total of fatty acid in triglyceride and free fatty acid) by gas chromatography. The vertical axis indicates the total fatty acid concentration (error bar indicates the standard deviation). The bottom right is a result of measuring oil content corresponding to a normal hexane value with an oil content measuring reagent kit. The vertical axis indicates the ratio of residual oil content when the measurement value at 0 hours is considered as 100% (error bar indicates the standard deviation).

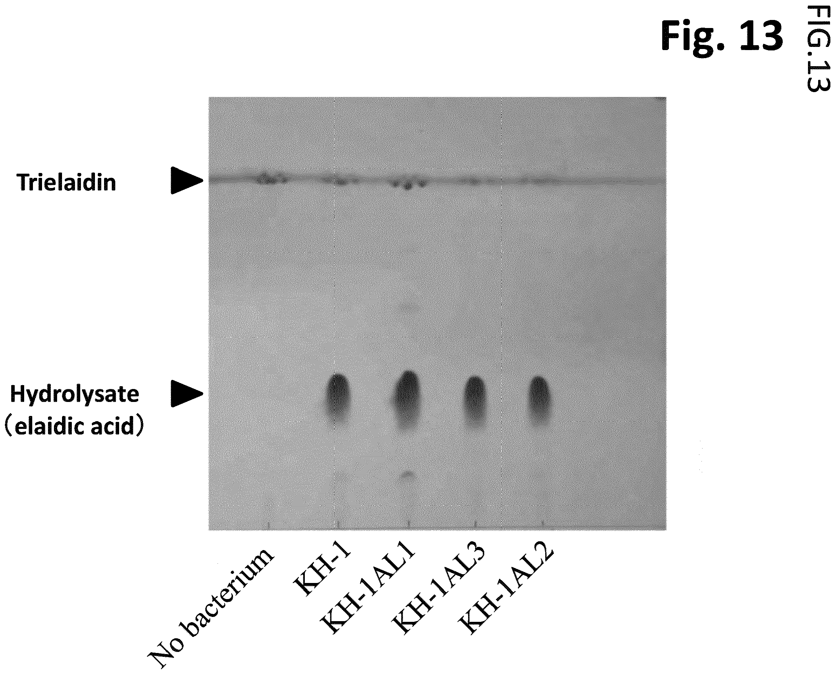

[Figure 13] Figure **13** is a picture of TLC analysis showing decomposition of trielaidin with culture supernatant of KH-1, KH-1AL1, KH-1AL2, and KH-1AL3 in 28°C culture. The picture shows, from the left, results for no bacterium (control), KH-1, KH-1AL1, KH-1AL3, and KH-1AL2.

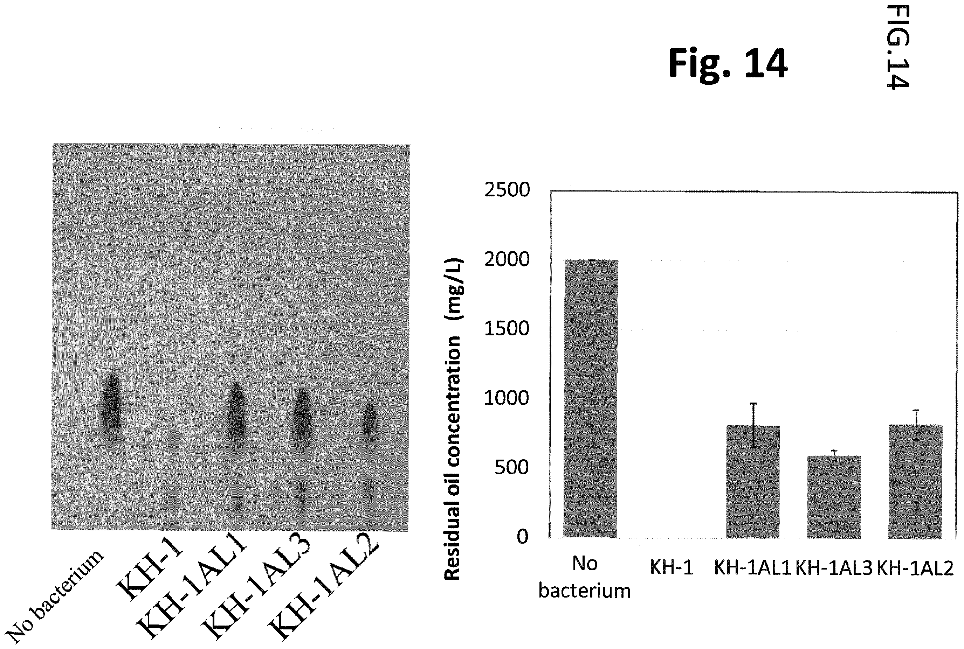

[Figure 14] Figure **14** shows assimilation and decomposition of elaidic acid by KH-1, KH-1AL1, KH-1AL2, and KH-1AL3 in 28°C culture. The left side is a picture of TLC analysis showing residual elaidic acid in the culture. The right side is a result of measuring oil content corresponding to a normal hexane value with an oil content measuring reagent kit. The vertical axis indicates the residual oil content concentration (mg/L). The columns indicate, from the left, results for no bacterium (control), KH-1, KH-1AL1, KH-1AL3, and KH-1AL2 (error bar indicates the standard deviation).

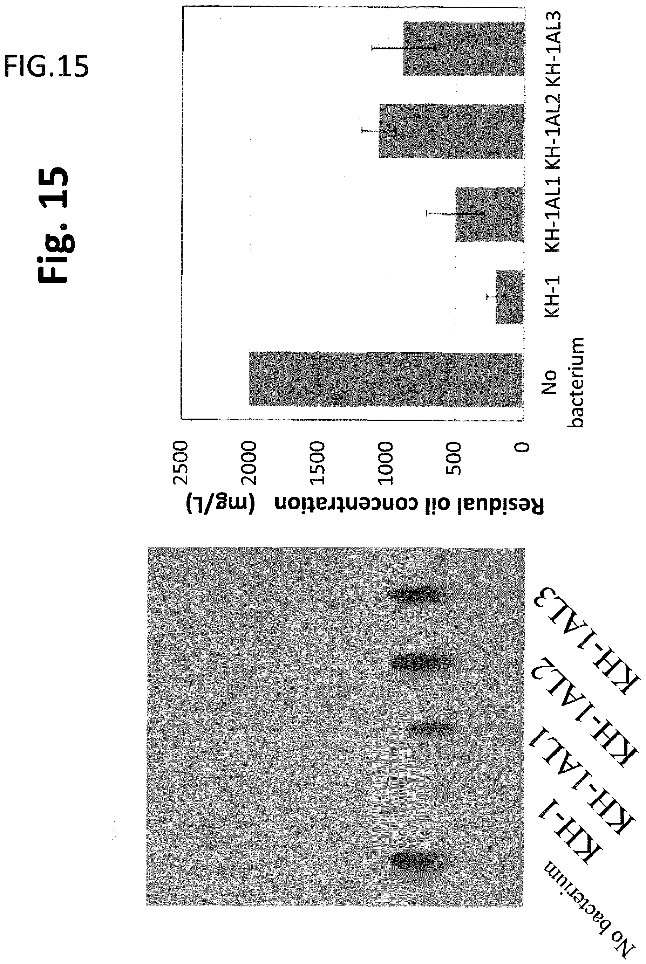

[Figure 15] Figure **15** shows assimilation and decomposition of elaidic acid by KH-1, KH-1AL1, KH-1AL2, and KH-1AL3 in 15°C culture. The left side is a picture of TLC analysis showing residual elaidic acid in the culture. The right side is a result of measuring oil content corresponding to a normal hexane value with an oil content measuring reagent kit. The vertical axis indicates the residual oil content concentration (mg/L). The columns indicate, from the left, results for no bacterium (control), KH-1, KH-1AL1, KH-1AL2, and KH-1AL3 (error bar indicates the standard deviation).

### [Description of Embodiments]

The present disclosure is described hereinafter while showing the best mode thereof. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present invention pertains. In case of a contradiction, the present specification (including the definitions) takes precedence.

The definitions of the terms and/or basic technical concepts that are particularly used herein are described hereinafter when appropriate.

### (Definitions, etc.)

As used herein, "lipase" refers to an enzyme, which is a type of esterase and reversibly catalyzes a reaction that hydrolyzes and decomposes neutral fat (glycerol ester) into fatty acid and glycerol. Examples of lipase include triglycerol lipase classified by an enzyme commission number (EC number) as EC 3.1.1.3.

As used herein, "oil and fat" refers to an oily substance, including an ester group-containing compound formed by a dehydration condensation between a hydroxyl group-containing compound and fatty acid. While the hydroxyl group-containing compound is typically glycerin, other examples thereof include polyglycerin and the like. As used herein, an ester group-containing compound formed by a dehydration condensation between glycerin and fatty acid is referred to as "glyceride" in the same manner as the meaning that is generally used in the art. If the hydroxyl group-containing compound has a plurality of hydroxyl groups, the compound falls under the ester group-containing compound herein as long as at least one of the hydroxyl groups forms an ester by a dehydration condensation with a fatty acid.

For example, kitchen wastewater produced by the restaurant industry, wastewater from a food factory, or the like contains oil and fat. In view of grease traps and dissolved air flotation apparatuses that are treatment facilities for removing oil and fat by solid-liquid separation being a source of foul odor or pests, cost and labor associated with maintenance such as collection and transport of the separated oil and cleaning, cost of coagulants, and the like, the microorganism or composition of the present disclosure is used for eliminating oil within a grease trap or factory wastewater treatment facility.

The present disclosure provides a microbial formulation for a grease trap and for factory wastewater comprising the oil and fat decomposing bacteria of the present disclosure. When applied particularly to factory wastewater, the utilization ratio of a dissolved air flotation apparatus can be reduced or even replaced. Food factory wastewater contains trans fatty acid, which is not ultimately removed in many cases and can be a problem as a residual contamination. Meanwhile, the microorganisms of the present disclosure can solve such a problem. Kitchen wastewater produced by the restaurant industry not only contains oil and fat at generally 1 g/L or greater and up to a high concentration of 10 g/L or greater, but also wastewater within many grease traps has a very short retention time of about 10 minutes. The microorganisms of the present disclosure can also be used in such an environment.

Food waste, animal farming waste, sludge from wastewater treatment plants, and the like also contain a large amount of oil and fat, many of which also contains trans fatty acid. While treatment of such solid waste often utilizes microorganisms, high oil content results in difficulty in treatment and oil content remaining. The microorganism or composition of the present disclosure can also be applied to decomposition treatment of oil content in such waste.

As used herein, "fatty acid" is a compound with 2 to 100 carbon atoms and at least one carboxyl group. Typically, a carbon chain in a fatty acid is linear, but can be branched or comprise a ring. Typically, a fatty acid comprises one carboxyl group, but can comprise a plurality of carboxyl groups. A carbon chain in a fatty acid can comprise a C=C double bond. "Trans fatty acid" is used in the meaning that is generally used in the art, referring to unsaturated fatty acid with a trans double bond. As used herein, "trans fatty acid-containing oil and fat" refers to a compound formed by dehydration condensation between trans fatty acid and hydroxyl group-containing compound. Trans fatty acid encompasses elaidic acid, vaccenic acid, and the like, but when mentioned herein, the type of trans fatty acid is not particularly limited. The ratio of trans fatty acid that is present in trans fatty acid-containing oil and fat is not particularly limited. "Trans" and "cis" configurations of double bonds are used in the meaning that is generally used in the art. The follow structure, where four substituents (R₁, R₂, R₃, and R₄) are bound to two carbon atoms forming a double bond 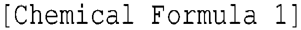 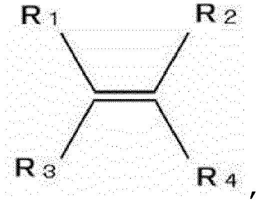 is referred to as a cis configuration if R₁ and R₂, or R₃ and R₄ are groups other than hydrogen, and the remaining two substituents are hydrogen atoms, and is referred to as a trans configuration if R₁ and R₄, or R₃ and R₂ are groups other than hydrogen, and the remaining two substituents are hydrogen atoms. Trans fatty acid is naturally present at a trace amount as conjugated linoleic acid or vaccenic acid. For example, fat content of ruminants contains a relatively large amount. Trans fatty acid can be generated in the hydrogenation process for manufacturing saturated fatty acid from unsaturated fatty acid and purification of vegetable oil containing a large amount of unsaturated fatty acid. For this reason, margarine, fat spread, shortening, and the like can contain a relatively large amount of trans fatty acid.

As used herein, "normal hexane (n-Hex) value" is the amount of nonvolatile substance extracted with normal hexane and refers to an indicator that indicates the amount of oil content (oil and fat, hydrolysate thereof, etc.) in water. An n-Hex value can be calculated, for example, in accordance with JIS K 0102. The n-Hex value can also be calculated by using a simple measuring reagent kit from measuring poly(N-isopropylacrylamide) extracted substances.

As used herein, "assimilation" refers to utilization as a nutrient source. The substance subjected to assimilation (e.g., oil and fat) is eliminated as a result.

As used herein, "decomposition", when used in the context of oil and fat and/or fatty acid, refers to subjected oil and fat and/or fatty acid becoming smaller molecules, such as being degraded into glycerol and (free) fatty acid. Conversion of fatty acid into fatty acid with fewer carbon atoms and conversion into carbon dioxide and water are also referred to as decomposition.

As used herein, "ability to assimilate trans fatty acid-containing oil and fat" refers to activity to assimilate trans fatty acid-containing oil and fat. As used herein, "assimilate trans fatty acid-containing oil and fat" is used in the meaning that is generally used in the art, referring to microorganisms taking in trans fatty acid-containing oil and fat as a nutrient source such as a carbon source. "Assimilate" also includes hydrolysis into glycerol and free fatty acid as well a change to a part of another substance. The ability to assimilate trans fatty acid-containing oil and fat can be measured and identified by the following tests:
*test for checking whether bacteria can grow in a medium comprising trans fatty acid-containing oil and fat as the sole carbon source;
*test for checking whether a colony is formed in a medium comprising trans fatty acid-containing oil and fat as the sole carbon source;
*test for measuring, with the growth, a decrease in the normal hexane value in the culture supernatant;
*test for measuring, with the growth, the total fatty acids (sum of fatty acid in oil and fat and free fatty acid) in the culture supernatant after conversion into methyl ester by gas chromatography; and
*test for measuring, with the growth, the amount of oil and fat and free fatty acid in culture supernatant by thin-layer chromatography.

The present specification provides more detailed individual measuring methods. Those skilled in the art can also perform such measurements by using any other equipment or condition.

As used herein, "ability to decompose trans fatty acid-containing oil and fat" refers to activity to hydrolyze trans fatty acid-containing oil and fat into glycerol and free fatty acid. The ability to decompose trans fatty acid-containing oil and fat can be measured and identified by the following tests:
*test for checking whether bacteria have the ability to assimilate trans fatty acid-containing oil and fat;
*test for determining the amount of oil and fat and free fatty acid in culture supernatant by thin-layer chromatography;
*test for culturing bacteria by providing trans fatty acid-containing oil and fat as a carbon source and measuring the amount of decrease in the normal hexane value in the culture supernatant;
*test for measuring the concentration of free fatty acid in the culture supernatant by gas chromatography;
*test for measuring the concentration of free fatty acid in the culture supernatant by GC-MS;
*test for measuring the concentration of free fatty acid in the culture supernatant by HPLC;
*test for checking whether a clear zone is observed around a colony formed on an agar medium containing trans fatty acid-containing oil and fat; and
*test for preparing a test water comprising trans fatty acid-containing oil and fat as the primary organic matter (e.g., 70% by weight or greater among all organic matters) and measuring the biochemical oxygen demand (BOD).

The present specification provides more detailed individual measuring methods. Those skilled in the art can also perform such measurements by using any other equipment or condition.

As used herein, "ability to assimilate trans fatty acid" refers to the ability to assimilate trans fatty acid. The ability to assimilate trans fatty acid can be measured and identified by the following tests:
*test for checking whether bacteria can grow in a medium comprising trans fatty acid as the sole carbon source;
*test for checking whether a colony is formed in a medium comprising trans fatty acid as the sole carbon source;
*test for measuring, with the growth, a decrease in the normal hexane value in the culture supernatant;
*test for measuring, with the growth, the trans fatty acid concentration in the culture supernatant by gas chromatography;
*test for measuring, with the growth, the trans fatty acid concentration in the culture supernatant by HPLC; and
*test for measuring, with the growth, the amount of trans fatty acid in the culture supernatant by thin-layer chromatography.

The present specification provides more detailed individual measuring methods. Those skilled in the art can also perform such measurements by using any other equipment or condition.

As used herein, "ability to decompose trans fatty acid" refers to the ability to decompose trans fatty acid. The ability to decompose trans fatty acid can be measured and identified by the following tests:
*test for checking whether bacteria have the ability to assimilate trans fatty acid;
*test for determining the amount of trans fatty acid in culture supernatant by thin-layer chromatography;
*test for culturing bacteria by providing trans fatty acid as a carbon source and measuring the decrease in the normal hexane value in the supernatant;

*test for measuring the concentration of trans fatty acid in the culture supernatant by gas chromatography;
*test for measuring the concentration of trans fatty acid in the culture supernatant by GC-MS;
*test for measuring the concentration of trans fatty acid in the culture supernatant by HPLC;
*test for checking whether a clear zone is observed around a colony formed in an agar medium containing trans fatty acid; and
*test for preparing a test waster comprising trans fatty acid as the primary organic matter (e.g., 70% by weight or greater among all organic mattes) and measuring the biochemical oxygen demand (BOD).

The present specification provides more detailed individual measuring methods. Those skilled in the art can also perform such measurements by using any other equipment or condition.

As used herein, "ability to decompose oil and fat at 15°C" refers to the activity to hydrolyze oil and fat into glycerol and free fatty acid at a low temperature. The ability to decompose oil and fat at 15°C can be measured and identified by the following tests. It is sufficient to show the ability to decompose in one of the following tests. Decomposition does not necessarily need to be observed in all of the tests.
*test for checking whether bacteria have the ability to assimilate oil and fat at 15°C;
*test for checking whether a clear zone is observed around a colony formed on an agar medium containing oil and fat at 15°C;
*test for culturing bacteria at 15°C by providing oil and fat as a carbon source and measuring the amount of decrease in the normal hexane value in the culture supernatant;

*test for culturing bacteria at 15°C by providing oil and fat as a carbon source and measuring the change over time in the amount of oil and fat and free fatty acid in the culture supernatant by thin-layer chromatography. The bacteria have the ability to decompose if the amount of oil and fat decreases with passage of time. Alternatively, the bacteria are considered as having the ability to decompose once the amount of free fatty acid increases;
*test for culturing bacteria at 15°C by providing oil and fat as a carbon source and measuring the concentration of free fatty acid in the culture supernatant by instrumental analysis such as gas chromatography, GC-MS, or HPLC. The bacteria are considered as having the ability to decompose once the amount of free fatty acid increases; and
*test for preparing a test water comprising oil and fat as the primary organic matter (e.g., 70% by weight or greater among all organic mattes) and measuring the biochemical oxygen demand (BOD).

The present specification provides more detailed individual measuring methods. Those skilled in the art can also perform such measurements by using any other equipment or condition.

As used herein, "oil treating component" refers to a component that assists in the assimilation and decomposition of oil and fat and/or fatty acid. Specific examples thereof include components that promote dispersion of oil and fat and/or fatty acid such as biosurfactants, components that decompose oil and fat into fatty acid and glycerol, components that decompose fatty acid, components that decompose glycerol, components that adsorbs to and remove oil from a subject of treatment, and the like. In one aspect, an oil treating component can comprise a biosurfactant produced by the microorganism of the present disclosure.

As used herein, "oil decomposing agent" refers to a formulation that is capable of decomposing oil and fat and/or fatty acid, comprising the microorganism of the present disclosure as an active ingredient. In the present disclosure, an oil decomposing agent can be used in combination with an oil treating component. The timing of combined use of an oil decomposing agent and an oil treating component in such a case can be simultaneous use, or one can be used before the other. An oil decomposing agent can further contain a component for enhancing the activity of the bacterial strain that is used or lipase derived from the bacterial strain (e.g., carbon source or nitrogen source), surfactant, dry protection agent, component for maintaining bacteria for a long period of time, antiseptic, excipient, reinforcing agent, antioxidant, or the like.

A "derivative strain", "similar strain", or "mutant strain" as used herein preferably, but is not intended to be limited to, comprise a gene (e.g., 16S rDNA) comprising a region that is substantially homologous to a DNA of the target microorganism. In various embodiments, such a strain has a full genomic sequence that is at least 30%, 40%, 50%, 60%, 70%, 80%, 90%, 95%, or 99% identical in comparison to the full genomic sequence of the original strain after alignment with a computer homology program that is known in the art. This refers to a microorganism altered by a mutation, substitution, deletion, and/or addition to a gene, wherein the derivative strain still exhibits the biological function of the original microorganism, but not necessarily to the same degree. For example, a gene mutation can be introduced by using any known mutagen, UV, plasma, or the like. In one embodiment, a "derivative strain", "similar strain", or "mutant strain" is a strain of the same genus and/or species as the original strain. For example, the biological function of such a microorganism can be examined by a suitable and available in vitro assay that is described herein or known in the art.

As used herein, "homology" of genes refers to the degree of identity of two or more genetic sequences with respect to one another, and having "homology" generally refers to having a high degree of identity or similarity. Therefore, the identity or similarity of sequences is higher when homology of two genes is high. Whether two types of genes have homology can be found by direct comparison of sequences or by a hybridization method under stringent conditions for nucleic acids. When two genetic sequences are directly compared, the genes are homologous typically if DNA sequences are at least 50% identical, preferably at least 70% identical, and more preferably at least 80%, 90%, 95%, 96%, 97%, 98%, or 99% identical between the genetic sequences. As used herein, "similarity" of gens or base sequences refers to the degree of similarity of two or more genetic sequences with one another and refers to a high degree of similarity with another sequence with identity. "Similarity" is a numerical value that takes into account similar bases in addition to identity into the calculation. In this regard, a similar base refers to instances with a partial match in a mixed base (e.g., R = A + G, M = A + C, W = A + T, S = C + G, Y = C + T, K = G + T, H = A + T + C, B = G + T + C, D = G + A + T, V= = A + C + G, N = A + C + G + T).

Amino acids may be mentioned herein by either their commonly known three letter symbols or their one character symbols recommended by the IUPAC-IUB Biochemical Nomenclature Commission. Similarly, nucleotides may be mentioned by their commonly recognized one character codes. Comparison of similarity, identity, and homology of an amino acid sequence and a base sequence is calculated herein by using a sequence analysis tool BLAST with default parameters. For example, identity can be searched using BLAST 2.7.1 (published on October 19, 2017) of the NCBI. Herein, values for identity generally refer to a value obtained when aligned under the default conditions using BLAST. However, when a higher value is obtained by changing a parameter, the highest value is considered the value of identity. When identity is evaluated in a plurality of regions, the highest value thereamong is considered the value of identity. "Similarity" is a value calculated by taking into consideration a similar amino acid in addition to identity.

In one embodiment of the present disclosure, the numerical value of identity or the like, i.e., "70% or greater", can be, for example, 70% or greater, 75% or greater, 80% or greater, 85% or greater, 90% or greater, 95% or greater, 96% or greater, 97% or greater, 98% or greater, 99% or greater, or 100% or greater, or within a range between any two of numerical values of such starting points. The "identity" described above computes the ratio of the number of homologous amino acids or bases in two or more amino acid or base sequences in accordance with a known method described above. Specifically, amino acid or base sequences in a group of amino acid or base sequences to be compared are aligned before computing the ratio, and a space is introduced in a part of the amino acid or base sequences if needed to maximize the ratio of identical amino acids or bases. A method for alignment, ratio computation method, comparison method, and computer program associated therewith are conventional and well known in the art (e.g., aforementioned BLAST or the like). As used herein, "identity" and "similarity" can be represented by a value measured by NCBI's BLAST, unless specifically noted otherwise. Blastp can be used with the default settings as the algorithm for comparing amino acid or base sequences with BLAST. Measurement results are quantified as Positives or Identities. When "similarity" is used instead of "identity" in such a case, the numerical value also takes into consideration those that fall under the definition of "similar" "amino acid" or "base" described herein.

As used herein, "biological function" refers to a specific function that a certain microorganism can have in the context of the microorganism. Examples thereof include, but are not limited to, decomposition of oil and fat (e.g., decomposition of trans fatty acid-containing oil and fat). In the present disclosure, examples thereof include, but are not limited to, decomposition of trans fatty acid-containing oil and fat as well as decomposition of cis fatty acid-containing oil and fat, decomposition of cis fatty acid, decomposition of saturated fatty acid-containing oil and fat, decomposition of saturated fatty acid, and the like. A biological function can be exerted by a corresponding "biological activity" herein. As used herein, "biological activity" refers to activity that a certain microorganism can have under a certain environment, and encompasses activities exerting various functions (e.g., activity to decompose trans fatty acid-containing oil and fat). Such a biological activity can be measured by technologies that are well known in the art. Therefore, "activity" refers to various measurable indicators that affect a response (i.e., has a measurable effect in response to some exposure or stimulation), and can include, for example, the amount of a protein upstream or downstream after some stimulation or event or other similar scales of function of the microorganism of the present disclosure.

As used herein, the "amount" of an analyte in a sample generally refers to an absolute value reflecting the mass of the analyte that can be detected in a volume of the sample. However, amount is also intended as a relative amount as compared to the amount of another analyte. For example, the amount of an analyte in a sample can be an amount that is greater than a control level or a normal level of an analyte that is generally present in the sample.

The term "about" refers to the indicated value plus or minus 10%.

As used herein, "kit" refers to a unit providing parts to be provided (e.g., composition comprising the microorganism of the present disclosure, additional component, buffer, user manual, and the like) which are generally separated into two or more segments. Such a kit form is preferred when providing a composition, which should not be provided in a mixed state for stability or the like and is preferably used by mixing immediately prior to use. Such a kit advantageously comprises an instruction or user manual describing how the provided parts (e.g., composition comprising a microorganism or addition component) or the like are used or how the parts should be processed. When a kit is used herein, the kit generally comprises an instruction or the like describing the method of use of the microorganism or composition of the present disclosure, or the like.

As used herein, "instruction" is a document with an explanation of the method of use of the present disclosure for users. The instruction provides description for instructing the method of use of the present disclosure. If required, the instruction is prepared in accordance with a format specified by a regulatory authority of the country in which the present disclosure is practiced (e.g., Ministry of Health, Labour and Welfare, Ministry of Agriculture, Forestry and Fisheries, or the like in Japan, Food and Drug Administration (FDA) or Department of Agriculture (USDA) in the U.S., or the like), with an explicit description showing approval by the regulatory authority. An instruction can be provided in, but not limited to, paper media. An instruction can also be provided in a form such as electronic media (e.g., web sites provided on the Internet or emails).

### (Preferred embodiments>

Preferred embodiments of the present disclosure are described below. Embodiments provided below are provided to facilitate the understanding of the present disclosure. It is understood that the scope of the present disclosure should not be limited to the following descriptions. Thus, it is apparent that those skilled in the art can make appropriate modifications within the scope of the present disclosure by referring to the descriptions herein. It is understood that the following embodiments of the present disclosure can be used alone or in combination.

### (Oil and fat decomposing microorganism)

In one aspect, the present disclosure provides a new microorganism with an ability to decompose oil and fat. In particular, the microorganism of the present disclosure has one or more features of the ability to assimilate trans fatty acid-containing oil and fat, ability to assimilate trans fatty acid, ability to decompose trans fatty acid, ability to decompose trans fatty acid-containing oil and fat, and/or ability to assimilate and/or decompose oil and fat and/or fatty acid at 15°C.

In one embodiment, the microorganism of the present disclosure is a bacterium of the family Burkholderiaceae. In one embodiment, the microorganism of the present disclosure is a bacterium of the genus Burkholderia. The genus of Burkholderia is a gram negative, non-spore forming aerobic bacillus with a polar flagella, and is the type genus of the family Burkholderiaceae. In one embodiment, the microorganism of the present disclosure is Burkholderia arboris, Burkholderia ambifaria, or Burkholderia cepacia, and is preferably Burkholderia arboris or Burkholderia ambifaria. In one embodiment, the microorganism of the present disclosure is a microorganism belonging to the Burkholderia cepacia complex. Burkholderia cepacia complex is a classification of microorganisms of the genus Burkholderia that are genetically very close, including ambifaria, anthina, arboris, cenocepacia, cepacia, contaminans, diffusa, dolosa, lata, latens, metallica, multivorans, pseudomultivorans, puraquae, pyrrocinia, seminalis, stabilis, stagnalis, territorii, ubonensis, and vietnamiensis (Martina P et al., Int J Syst Evol Microbiol. 2018 Jan; 68(1): 14-20.). In another embodiment, the Burkholderia bacteria of the present disclosure can be metallica, seminalis, anthina, ambifaria, diffusa, ubonensis, multivorans, latens, cenocepacia, vietnamiensis, pyrrocinia, stabilis, glumae, gladioli, plantarii, oklahomensis, thailandensis, mallei, pseudomallei, or phytofirmans. The inventors identified a new microorganism (KH-1) as Burkholderia arboris by base sequencing and phylogenetic analysis of 16S ribosome DNA. The bacteria were deposited with the National Institute of Technology and Evaluation, Patent Microorganisms Depositary and accepted on June 4, 2018, and a certificate of deposit was issued on June 12, 2018. The receipt number is NITE ABP-02731. The inventors also identified bacteria (KH-1AL1, KH-1AL2, and KH-1AL3) of the genus Burkholderia. The bacteria were deposited with the National Institute of Technology and Evaluation, Patent Microorganisms Depositary and accepted on June 28, 2019. The receipt numbers are NITE ABP-02977, NITE ABP-02978, and NITE ABP-02979, respectively. In one embodiment, the microorganism of the present disclosure is a KH-1 strain (bacterial strain identified by accession number NITE BP-02731/receipt number NITE ABP-02731), a KH-1AL1 strain (bacterial strain identified by receipt number NITE ABP-02977), a KH-1AL2 strain (bacterial strain identified by receipt number NITE ABP-02978), or a KH-1AL3 strain (bacterial strain identified by receipt number NITE ABP-02979) of a Burkholderia bacteria, or a derivative strain thereof.

In one embodiment, the microorganism of the present disclosure is a derivative strain of a KH-1 strain (bacterial strain identified by accession number NITE BP-02731/receipt number NITE ABP-02731), a KH-1AL1 strain (bacterial strain identified by receipt number NITE ABP-02977), a KH-1AL2 strain (bacterial strain identified by receipt number NITE ABP-02978), or a KH-1AL3 strain (bacterial strain identified by receipt number NITE ABP-02979) of a Burkholderia bacteria. In this regard, a derivative strain does not need to be a strain obtained based on a KH-1 strain, a KH-1AL1 strain, a KH-1AL2 strain, or a KH-1AL3 strain of a Burkholderia bacteria, and refers to a microorganism that exhibits a biological function of these Burkholderia bacteria strains, but not necessarily to the same degree. In one embodiment, a microorganism that is a derivative strain of the present disclosure exhibits a biological function selected from the group consisting of an ability to assimilate (decompose) oil and fat (e.g., trans fatty acid-containing oil and fat) and an ability to assimilate (decompose) trans fatty acid at a low temperature (e.g., 25°C or less, 20°C or less, 15°C or less, 10°C or less, 5°C or less, or the like) in the same manner as a Burkholderia bacteria KH-1 strain, KH-1AL1 strain, KH-1AL2 strain, or KH-1AL3 strain, but the degree of the biological function can be different from a KH-1 strain, KH-1AL1 strain, KH-1AL2 strain, or KH-1AL3 strain. In one embodiment, a microorganism that is a derivative strain of the present disclosure is a bacterium in the family Burkholderiaceae, more specifically a bacterium in the genus Burkholderia, and still more specifically a microorganism belonging to Burkholderia arboris, Burkholderia ambifaria, or Burkholderia cepacia complex.

The microorganisms of the present disclosure (including derivative strains of a KH-1 strain, KH-1AL1 strain, KH-1AL2 strain, or KH-1AL3 strain) can be isolatable on an inorganic salt agar medium, which comprises oil and fat as the sole carbon source and has a pH adjusted to 6 to 8. In one embodiment, the microorganisms of the present disclosure (including derivative strains of a KH-1 strain, KH-1AL1 strain, KH-1AL2 strain, or KH-1AL3 strain) can be differentiable by checking for formation of a clear zone (hollow) around a colony appeared on an agar medium.

In one embodiment, the microorganisms of the present disclosure (including derivative strains of a KH-1 strain, KH-1AL1 strain, KH-1AL2 strain, or KH-1AL3 strain) have an ability to assimilate trans fatty acid or trans fatty acid-containing oil and fat. In one embodiment, the microorganisms of the present disclosure (including derivative strains of a KH-1 strain, KH-1AL1 strain, KH-1AL2 strain, or KH-1AL3 strain) have an ability to decompose trans fatty acid. In one embodiment, the microorganisms of the present disclosure (including derivative strains of a KH-1 strain, KH-1AL1 strain, KH-1AL2 strain, or KH-1AL3 strain) have an ability to decompose trans fatty acid-containing oil and fat.

In one embodiment, the microorganisms of the present disclosure (including derivative strains of a KH-1 strain, KH-1AL1 strain, KH-1AL2 strain, or KH-1AL3 strain) have an ability to decompose oil and fat and/or fatty acid at 15°C. In one embodiment, the microorganisms of the present disclosure (including derivative strains of a KH-1 strain, KH-1AL1 strain, KH-1AL2 strain, or KH-1AL3 strain) retain the ability to assimilate or decompose trans fatty acid or trans fatty acid-containing oil and fat at 15°C.

In one embodiment, the microorganisms of the present disclosure (including derivative strains of a KH-1 strain, KH-1AL1 strain, KH-1AL2 strain, or KH-1AL3 strain) can secrete a biosurfactant when cultured in a medium comprising oil and fat or fatty acid.

In one embodiment, the microorganisms of the present disclosure (including derivative strains of a KH-1 strain, KH-1AL1 strain, KH-1AL2 strain, or KH-1AL3 strain) have an ability to decompose oil and fat so that oil content corresponding to a normal hexane value in the supernatant would be less than 9 g/L, less than 8 g/L, less than 7 g/L, less than 6 g/L, less than 5 g/L, less than 4 g/L, less than 3 g/L, less than 2 g/L, less than 1 g/L, less than 0.7 g/L, less than 0.5 g/L, less than 0.2 g/L, less than 0.1 g/L, less than 0.07 g/L, less than 0.05 g/L, less than 0.02 g/L, or less than 0.01 g/L after 24 or 48 hours culture wherein the microorganisms are inoculated into an inorganic salt medium comprising 10 g/L of canola oil at a density that would result in a final concentration of OD₆₆₀ = 0.03 or 0.02 and cultured at a pH of 7.0 and 15°C under 200 ml/min of air circulation. In particular, the microorganisms of the present disclosure (including derivative strains of a KH-1 strain) preferably have an ability to decompose oil and fat that reduces oil content corresponding to a normal hexane value to less than about 6 g/L when inoculated at a density of OD₆₆₀ = 0.02 and cultured for 48 hours when determined under this condition, and more preferably have an ability to decompose oil and fat that reduces oil content corresponding to a normal hexane value to less than 6 g/L when inoculated at a density of OD₆₆₀ = 0.03 and cultured for 24 hours. A microorganism having such an ability to decompose oil and fat at a low temperature can be advantageously used in various applications of the present disclosure.

In one embodiment, the microorganisms of the present disclosure (including derivative strains of a KH-1 strain, KH-1AL1 strain, KH-1AL2 strain, or KH-1AL3 strain) can have an ability to decompose oil and fat for assimilating and/or decomposing trans fatty acid-containing oil and fat in a 15°C or 28°C medium with a pH of 7.0 comprising trans fatty acid (e.g., elaidic acid, palmitelaidic acid, or vaccenic acid)-containing oil and fat.

In one embodiment, the microorganisms of the present disclosure (including derivative strains of a KH-1 strain, KH-1AL1 strain, KH-1AL2 strain, or KH-1AL3 strain) have an ability to decompose trans fatty acid so that the elaidic acid concentration in the supernatant would be less than 0.15%, less than 0.12%, less than 0.1%, less than 0.09%, less than 0.08%, less than 0.07%, less than 0.06%, less than 0.05%, less than 0.04%, less than 0.03%, less than 0.02%, less than 0.015%, less than 0.01%, less than 0.007%, less than 0.005%, less than 0.002%, less than 0.001%, less than 0.0007%, less than 0.0005%, less than 0.0002%, or less than 0.0001% in terms of % by weight after 72 hours culture wherein the microorganisms are inoculated into an inorganic salt medium comprising Triton X-100 and elaidic acid at concentrations of 0.25% by weight and 0.2% by weight, respectively, at a density that would result in a final concentration of OD₆₆₀ = 0.08 and cultured at a pH of 7.0 and 15°C. The microorganisms of the present disclosure (including derivative strains of a KH-1 strain, KH-1AL1 strain, KH-1AL2 strain, or KH-1AL3 strain) preferably have an ability to reduce the elaidic acid concentration in the supernatant to less than 0.15%, less than 0.12%, less than 0.1%, or less than 0.05%, especially less than 0.12% when determined under this condition. A microorganism having such an ability to decompose trans fatty acid can be advantageously used in various applications of the present disclosure.

In one embodiment, the microorganisms of the present disclosure (including derivative strains of a KH-1 strain, KH-1AL1 strain, KH-1AL2 strain, or KH-1AL3 strain) have an ability to decompose trans fatty acid so that the elaidic acid concentration in the supernatant would be less than 0.15%, less than 0.12%, less than 0.1%, less than 0.09%, less than 0.08%, less than 0.07%, less than 0.06%, less than 0.05%, less than 0.04%, less than 0.03%, less than 0.02%, less than 0.015%, less than 0.01%, less than 0.007%, less than 0.005%, less than 0.002%, less than 0.001%, less than 0.0007%, less than 0.0005%, less than 0.0002%, or less than 0.0001% in terms of % by weight after 24 hours culture wherein the microorganisms are inoculated into an inorganic salt medium comprising Triton X-100 and elaidic acid at concentrations of 0.25% by weight and 0.2% by weight, respectively, at a density that would result in a final concentration of OD₆₆₀ = 0.04 and cultured at a pH of 7.0 and 28°C. The microorganisms of the present disclosure (including derivative strains of a KH-1 strain, KH-1AL1 strain, KH-1AL2 strain, or KH-1AL3 strain) preferably have an ability to reduce the elaidic acid concentration in the supernatant to less than 0.15%, less than 0.12%, less than 0.1%, or less than 0.05%, less than 0.02%, or less than 0.01%, especially less than 0.1% when determined under this condition. A microorganism having such an ability to decompose trans fatty acid can be advantageously used in various applications of the present disclosure.

In one embodiment, the microorganisms of the present disclosure (including derivative strains of a KH-1 strain, KH-1AL1 strain, KH-1AL2 strain, or KH-1AL3 strain) have an ability to decompose oil and fat so that oil content corresponding to a normal hexane value in the supernatant would be 1000% or less, 800% or less, 600% or less, 400% or less, 200% or less, 150% or less, 100% or less, 80% or less, 60% or less, 40% or less, 20% or less, 10% or less, or 5% or less after 24 hours in 15°C culture in comparison to the oil content corresponding to a normal hexane value in the supernatant after 24 hours in 28°C culture wherein the microorganisms are inoculated into an inorganic salt medium comprising 10 g/L of canola oil at a density that would result in a final concentration of OD₆₆₀ = 0.03 and cultured under 200 ml/min of air circulation at a pH of 7.0 and 15°C and 28°C, respectively. The microorganisms of the present disclosure (including derivative strains of a KH-1 strain) preferably have an ability to decompose oil and fat so that the ratio of residual oil and fat in 15°C culture would be 800% or less, 700% or less, 600% or less, 500% or less, 400% or less, and especially 700% or less in comparison to 28°C culture when determined under this condition. A microorganism having such an ability to decompose oil and fat at a low temperature can be advantageously used in various applications of the present disclosure.

In one embodiment, the microorganisms of the present disclosure (including derivative strains of a KH-1 strain, KH-1AL1 strain, KH-1AL2 strain, or KH-1AL3 strain) have an ability to decompose oil and fat so that the rate of decomposing total fatty acids would be 1000% or greater, 800% or greater, 600% or greater, 400% or greater, 200% or greater, 150% or greater, 100% or greater, 80% or greater, 60% or greater, 50% or greater, 40% or greater, 30% or greater, 20% or greater, 10% or greater, or 5% or greater in 15°C culture in comparison to 28°C culture wherein the microorganisms are inoculated into an inorganic salt medium comprising 10 g/L of canola oil at a density that would result in a final concentration of OD₆₆₀ = 0.03 and culture is started under 200 ml/min of air circulation at a pH of 7.0 and 15°C and 28°C,respectively. The microorganisms of the present disclosure (including derivative strains of a KH-1 strain) preferably have an ability to decompose oil and fat so that the rate of decomposing total fatty acids in 15°C culture would be 50% or greater, 40% or greater, 30% or greater, 20% or greater, or 10% or greater, especially 30% or greater in comparison to 28°C culture when determined under this condition. A microorganism having such an ability to decompose oil and fat at a low temperature can be advantageously used in various applications of the present disclosure.

In one embodiment, the ability to decompose/assimilate oil and fat and fatty acid of a microorganism can be evaluated by quantifying fatty acid contained in oil and fat and free fatty acid resulting from decomposition remaining in a culture by gas chromatography. The specific procedure for quantification involves firstly acidifying 1 mL of culture supernatant with hydrochloric acid and adding 2 mL of chloroform. After agitating for 2 minutes, the mixture is centrifuged, and 1 mL of the chloroform layer is transferred to another vessel and concentrated by evaporating the solvent. 2 mL of methanolysis solution (methanol: sulfuric acid = 17:3) is added and the mixture is heated for 2 hours at 100°C to methyl-esterify oil and fat and free fatty acid. Subsequently, 2 mL of chloroform and 1 mL of pure water are added and agitated, then the chloroform layer is analyzed by gas chromatography to quantify methyl ester of all fatty acids.

In one embodiment, the ability to decompose/assimilate oil and fat and fatty acid of a microorganism can be evaluated by analyzing oil and fat and fatty acid that is the decomposition product remaining in a medium by thin-layer chromatography (TLC). Specific procedure involves firstly adding chloroform at an equal amount to the culture supernatant to extract oil and fat. 5 µl of the extract is developed on a silica gel coated plate by using a development solvent comprising chloroform, acetone, and methanol at a ratio of 96:4:1 by volume. The plate is treated with molybdic acid n-hydrate to color oil and fat and/or fatty acid.

In one embodiment, the ability to decompose/assimilate oil and fat and fatty acid of a microorganism can be evaluated by studying the ability to grow in a medium having each of them as the sole carbon source.

In one embodiment, the microorganisms of the present disclosure (including derivative strains of a KH-1 strain, KH-1AL1 strain, KH-1AL2 strain, or KH-1AL3 strain) can have the ability to secrete lipase, but do not need to have the ability to secrete lipase.

In one embodiment, the microorganisms of the present disclosure (including derivative strains of a KH-1 strain, KH-1AL1 strain, KH-1AL2 strain, or KH-1AL3 strain) can be capable of growing/decomposing oil and fat under a weak acidic condition (e.g., pH of about 5.5 to 6.0).

In one embodiment, the ability of a microorganism to grow can be found by a method of measuring the absorbance (turbidity) at 660 nm (OD₆₆₀) as the bacterial optical density, a method of measuring colony forming unit (CFU), or the like. The latter spreads a certain amount of the undiluted and diluted solutions of culture on an agar medium and counts the colonies formed by stationary culture.

In one embodiment, the microorganism of the present disclosure can secrete lipase. The ability to secrete lipase of a microorganism can be evaluated by measuring lipase activity of culture supernatant obtained by centrifugation of the microorganism culture. Lipase activity can be determined by performing an enzymatic reaction using 4-nitrophenyl palmitate (4-NPP), which is an ester of palmitic acid and 4-nitrophenol, as the substrate and determining the amount of 4-nitrophenol resulting from hydrolysis of the ester by measuring an absorbance of 410 nm. Firstly, 4-NPP (18.9 mg) is added to 3% (v/v) Triton X-100 (12 ml) and dissolved at 70°C to prepare a substrate solution. 1 mL of the substrate solution, 0.9 mL of ion exchange water, and 1 mL of 150 mM GTA buffer (which is prepared by adding NaOH or HCl to 150 mM 3,3-dimethylglutaric acid, 150 mM Tris, and 150 mM 2-amino-2-methyl-1,3-propanediol and adjusting its pH to 7) are placed in a cell and incubated for 5 minutes at 28°C. 0.1 mL of culture supernatant is added thereto, and the value at 410 nm is measured while agitating.

Those skilled in the art can test a derivative strain of a KH-1 strain, KH-1AL1 strain, KH-1AL2 strain, or KH-1AL3 strain by suitably using the measurement method described above to obtain a derivative strain having the biological function (or the extent thereof) described above.

### (Composition comprising a microorganism)

In one aspect, the present disclosure provides a composition comprising the microorganism of the present disclosure. In one aspect, the present disclosure provides a composition comprising a culture supernatant of the microorganism of the present disclosure. The microorganism of the present disclosure can be manufactured by culturing with any suitable method. In one embodiment, a composition is an oil decomposing agent. In one embodiment, a composition is a fatty acid decomposing agent. In one embodiment, a compound comprising fewer carbons than the number of carbons contained in fatty acid can be produced by treatment with the fatty acid decomposing agent of the present disclosure. In one embodiment, a composition is a trans fatty acid decomposing agent.

### (Subject of application)

In one embodiment, examples of oil and fat to which the oil decomposing agent of the present disclosure is applied include, but are not limited to, vegetable oil and fat (cottonseed oil, rapeseed oil, soybean oil, corn oil, olive oil, safflower oil, rice oil, sesame oil, palm oil, coconut oil, peanut oil, and the like), animal oil and fat (lard, beef tallow, milk fat, and the like), fish oil, processed products of such oil and fat (margarine, shortening, butter, and the like), insulating oil, lubricating oil, and the like. Oil and fat can be in a form of an emulsion or in a free state.

In a specific embodiment, oil and fat to which the oil decomposing agent of the present disclosure is applied is oil and fat comprising trans fatty acid. Examples of such oil and fat include, but are not limited to, processed products such as oil and fat manufactured by hydrogenation (margarine, shortening, butter, and the like). Addition of hydrogen reduces the number of double bonds in unsaturated fatty acid and increases the ratio of saturated fatty acid, but can result in the generation of trans fatty acid. It is understood that margarine, fat spread and shortening manufactured by hydrogenation, bread, cakes, donuts, other confectionary, fried food, and the like using them as an ingredient, and the like contain trans fatty acid. Oil extracted from plants or fish is treated at a high temperature to eliminate undesirable odor in the purification step of the oil. During this step, trans fatty acid is produced from cis unsaturated fatty acid contained in oil, so that purified vegetable oil such as salad oil is also understood to contain a trace amount of trans fatty acid.

The subject to which the oil decomposing agent or fatty acid decomposing agent of the present disclosure is applied is not particularly limited. Examples thereof include, but are not limited to, industrial wastewater, domestic wastewater, industrial waste, domestic waste (food waste and the like), animal farming waste, aquaculture farms (and wastewater therefrom), animal sheds (and wastewater therefrom), slaughterhouses (and wastewater therefrom), soil contaminated with oil and fat, water contaminated with oil and fat (ocean, pond, river, animal drinking water, etc.), body surfaces of an animal, water tanks (for aquaculture, aquarium hobby, and the like), any oil and fat contaminated product (dishes, machine parts, and the like), grease traps installed in the kitchen or the like, fatbergs, drain pipes, insulating oil leaking from a transformer or the like, deteriorated insulating oil, and the like. Any subject thereamong can contain trans fatty acid-containing oil and fat and/or trans fatty acid, so that the oil decomposing agent or fatty acid decomposing agent of the present disclosure can be suitably applied. A "grease trap" is an apparatus for separating and collecting oil in wastewater, typically comprised of three tanks. The first tank comprises a basket and captures fragments of food ingredients, leftover food, and the like. The second tank separates oil from water. Wastewater separated from oil is sent to the third tank, where precipitating trash and the like is removed. Installation of a grease trap is mandatory for industrial kitchens in restaurants, hospitals, hotels, and the like. When applied to a grease trap, a decomposition/treatment tank can be provided separately, but an oil and fat decomposing agent or microorganism can be directly added to the grease trap for decomposition/treatment within the grease trap.

Since the microorganism of the present disclosure is highly efficient in treatment at a low temperature, there can be embodiments where low temperature treatment is desirable. For example, subjects for which treatment at less than 20°C (e.g., 15°C) is envisioned such as industrial wastewater, domestic wastewater, industrial waste, domestic waste (food waste and the like), soil contaminated with oil and fat, and water contaminated with oil and fat (ocean, pond, river, animal drinking water, and the like) are preferred examples of subjects to be treated by the present disclosure.

Specifically, a formulation or the like is administered or added, or a carrier with the microorganism of the present disclosure immobilized thereon or the like is installed within a wastewater path, wastewater storage tank, grease trap, or the like. A dedicated decomposition treatment tank can also be provided separately outside the grease trap.

In one embodiment, examples of wastewater include, but are not limited to, wastewater from restaurants, hospitals, hotels, or the like, domestic wastewater, industrial wastewater discharged from a food processing factory or oil and fat processing factory, and the like.

### (Usage form)

Examples of forms of the microorganism or composition of the present disclosure include liquid forms, solid forms, and the like. Examples of microorganisms or compositions in a liquid state include microorganism culture, microorganisms harvested from culture by centrifugation or the like and re-dispersed in water, buffer, or culture medium, and the like. Examples of microorganisms or compositions in a solid state include those that are dehydrated by centrifugation, press compression, or the like, those in a paste/mayonnaise-like state between a solid and liquid, dried forms that have been dried (e.g., vacuum drying or lyophilization). Examples of solid forms include powder, granules, tablets, and the like. A composition can also be provided in a state where a microorganism or a culture supernatant is immobilized on a carrier.

In one embodiment, the microorganism or composition of the present disclosure can be added to a liquid so that the density would be about 1 × 10⁸ cells/mL, about 1 × 10⁷ cells/mL, about 1 × 10⁶ cells/mL, about 1 × 10⁵ cells/mL, about 1 × 10⁴ cells/mL, about 1 × 10³ cells/mL, about 1 × 10² cells/mL, or about 10 cells/mL.

### (Applicable environment)

The microorganism or composition of the present disclosure can be used in any suitable environment.

In one embodiment, the microorganism or composition of the present disclosure can be used in an environment of 0 to 100°C, 5 to 70°C, 10 to less than 50°C, 15 to 40°C, 20 to 35°C, less than 70°C, less than 60°C, less than 50°C, less than 40°C, less than 30°C, less than 25°C, less than 20°C, less than 15°C, less than 10°C, less than 5°C, less than 0°C, about 70°C, about 60°C, about 50°C, about 40°C, about 30°C, about 25°C, about 15°C, about 10°C, about 5°C, or about 0°C.

In one embodiment, the microorganism or composition of the present disclosure can be used in an environment with a pH of 3 to 13, pH of 4 to 12, pH of 5 to 11, pH of 6 to 10, pH of 7 to 9, pH of 5.5 to 8.5, pH of about 3, pH of about 4, pH of about 5, pH of about 6, pH of about 7, pH of about 8, pH of about 9, pH of about 10, pH of about 11, pH of about 12, or pH of about 13.

In one embodiment, the microorganism or composition of the present disclosure can be used in an environment with a dissolved oxygen concentration (DO) of 0.05 mg/L or greater, 0.1 mg/L or greater, 0.5 mg/L or greater, or 1 mg/L or greater.

In one embodiment, the microorganism or composition of the present disclosure can be used in wastewater with a normal hexane value of 100 to 40000 mg/L, 200 to 30000 mg/L, or 300 to 30000 mg/L. While solid waste (may contain water) from food waste treatment, a slurry of sludge, or the like can have a higher concentration of oil and fat, the microorganism or composition of the present disclosure can also be applied usefully on such solid waste in one embodiment.

In one embodiment, the microorganism or composition of the present disclosure can be added to a subject comprising trans fatty acid at 50% by weight or greater, 20% by weight or greater, 10% by weight or greater, 7% by weight or greater, 5% by weight or greater, 2% by weight or greater, 1% by weight or greater, 0.7% by weight or greater, 0.5% by weight or greater, 0.2% by weight or greater, 0.1% by weight or greater, 0.07% by weight or greater, 0.05% by weight or greater, 0.02% by weight or greater, 0.01% by weight or greater, 0.007% by weight or greater, 0.005% by weight or greater, 0.002% by weight or greater, or 0.001% by weight or greater.

In one embodiment, the microorganism or composition of the present disclosure can be added to a subject with a ratio of trans fatty acid (total of free fatty acid and fatty acid in ester group-containing compound) in the contained oil and fat of 50% by weight or greater, 20% by weight or greater, 10% by weight or greater, 7% by weight or greater, 5% by weight or greater, 2% by weight or greater, 1% by weight or greater, 0.7% by weight or greater, 0.5% by weight or greater, 0.2% by weight or greater, 0.1% by weight or greater, 0.07% by weight or greater, 0.05% by weight or greater, 0.02% by weight or greater, 0.01% by weight or greater, 0.007% by weight or greater, 0.005% by weight or greater, 0.002% by weight or greater, or 0.001% by weight or greater.

In one embodiment, nitrogen can be present in a subject to which the microorganism or composition of the present disclosure is added in a form that is available to the microorganism, preferably in a form comprising ammonium salt, nitric acid salt, sulfuric acid salt, or an organic nitrogen compound, more preferably ammonium sulfate, urea, amino acid, or a peptide such as peptone, tryptone, or casamino acid. The amount of nitrogen that is present can be in the range of C/N = 2 to 50 (wherein C refers to carbon only from n-Hex), preferably C/N = 2 to 30, and more preferably C/N = 2 to 20, provided that C/N is a weight ratio of n-Hex derived carbon atoms to nitrogen atoms contained in wastewater. In one embodiment, nitrogen can be further added to attain such a range.

In one embodiment, phosphorous (P) can be present in a subject to which the microorganism or composition of the present disclosure is added in a form that is available to the microorganism, preferably in a form of phosphoric acid salt or nucleic acid, more preferably phosphoric acid salt. The amount of phosphorous that is present can be an amount at which N/P = 1 to 20 with respect to nitrogen, provided that N/P is the weight ratio of nitrogen atoms to phosphorous atoms contained in wastewater. In one embodiment, phosphorous can be further added to attain such a range.

In one embodiment, the microorganism or composition of the present disclosure can be used under a condition with a salt, surfactant, light, electric current, agitating, aeration, or any combination thereof.

In one embodiment, the microorganism or composition of the present disclosure can be applied after removing a substance (chlorine, antibiotic, or the like) that kills and suppresses the growth of the microorganism of the present disclosure.

In one embodiment, the microorganism or composition of the present disclosure can be used with a carrier on which the microorganism can be immobilized. Washout can be effectively avoided by using such a carrier. The material of a carrier is not particularly limited, as long as a microorganism can be immobilized. Examples thereof include carbon fiber (PAN based, pitch based, phenol resin based, and the like), polyethylene resin, polypropylene resin, polyurethane resin, polystyrene resin, polyvinyl chloride resin, polyvinyl acetate resin, polyvinyl alcohol resin, polyethylene glycol resin, acrylic resin, gelatin, sodium alginate, carrageenan, dextran, ceramics, silicon, metal, charcoal, activated carbon, minerals (zeolite, diatomaceous earth, and the like), composites thereof, and the like. It is preferable to use a porous or fibrous carrier to increase the rate of immobilization of microorganisms and the efficacy of the microorganisms. A microorganism can also be encapsulated in a gel-like carrier. Examples of shapes of a carrier include cubic, cuboidal, cylindrical, spherical, discoidal, sheet-like, membrane-like, and the like. For techniques of immobilizing microorganisms, see, for example, "Biseibutsu Koteika Ho niyoru Haisui Shori [Wastewater treatment by microorganism immobilization method], (Edited and authored by Ryuichi Sudo, The Industrial Water Institute)", "Biseibutsu Koteika Ho niyoru Mizushori - Tantai Koteika Ho Hokatsu Koteika Ho, Seibutsu Kasseitan Ho (Atarashii Mizushori Shiriizu (1) [Water treatment by microorganism immobilization method - carrier immobilization method and comprehensive immobilization method, Biological activated carbon method (New water treatment series (1)]) (Kazuhiro Mochizuki, Katsutoshi Hori, Hideki Tachimoto (authors), NTS Inc.)", or the like.

### (Additional components)

In one embodiment, the microorganism or composition of the present disclosure can be used in combination with an additional component. In one embodiment, an additional component can be added to a composition, or used separately from a microorganism or composition. When used separately, components can be provided as a kit.

In one embodiment, examples of the additional component include, but are not limited to, components elevating the activity of the microorganism that is used (e.g., carbon source or nitrogen source), surfactants, dry protection agents, components for maintaining a microorganism for a long period of time, antiseptics, excipients, reinforcing agents, antioxidants, another microorganism, and the like. Any suitable component can be used.

In one embodiment, examples of another microorganism include microorganisms with the ability to decompose oil and fat, microorganisms that produce lipase, microorganisms that decompose (assimilate) fatty acid and/or glycerol (which is a product of decomposition by lipase of oil and fat), microorganisms that decompose (assimilate) proteins, amino acids, nucleic acids, or polysaccharides (e.g., cellulose), and the like. Another microorganism is preferably capable of coexisting with the microorganism of the present disclosure.

A microorganism that produces lipase is eubacteria, yeast, filamentous eubacteria, or the like, preferably eubacteria or yeast, more preferably gram positive bacteria or proteobacteria. Examples of eubacteria that can be used include bacteria in the genus Bacillus, bacteria in the genus Corynebacterium, bacteria in the genus Rhodococcus, bacteria in the genus Burkholderia, bacteria in the genus Acinetobacter, bacteria in the genus Pseudomonas, bacteria in the genus Alcaligenes, bacteria in the genus Rhodobacter, bacteria in the genus Ralstonia, bacteria in the genus Acidovorax, bacteria in the genus Serratia, bacteria in the genus Flavobacterium, and the like. Examples of proteobacteria that can be used include alpha bacteria, beta bacteria, and gamma bacteria.

Examples of microorganisms that decompose (assimilate) glycerol that can be used include eubacteria, yeast, and filamentous eubacteria. Preferably, yeast in the genus Candida is used. Specific examples of yeast in the genus Candida include the Candida cylindracea SL1B2 strain (deposited with the National Institute of Technology and Evaluation, Patent Microorganisms Depositary under accession number NITE P-714). The bacterial strain has a property of being capable of coexisting with Burkholderia arboris in addition to having an excellent ability to assimilate glycerol. Thus, combined use of the Candida cylindracea SL1B2 strain is particularly preferable in an embodiment using Burkholderia arboris. Concomitant use of microorganisms that decompose (assimilate) glycerol can prevent a decrease in the rate of decomposing oil and fat due to accumulation of glycerol and make it possible to achieve an even more efficient decomposition of oil and fat.

A microorganism that decomposes (assimilates) fatty acid can be a eubacteria, yeast, filamentous eubacteria, or the like, preferably eubacteria or yeast, and more preferably yeast. Examples of yeast include yeast in the genus Yarrowia, yeast in the genus Cryptococcus, yeast in the genus Trichosporon, and yeast in the genus Hansenula.

### (Method of using microorganism)

In one aspect, the present disclosure provides a method of decomposing and removing oil and fat and/or fatty acid, comprising causing the microorganism or composition of the present disclosure to act on a subject of treatment. The subject of treatment can comprise trans fatty acid or trans fatty acid-containing oil and fat. A subject of treatment can be any subject of treatment described herein to which the microorganism or composition of the present disclosure can be applied. The method of decomposing and removing oil and fat and/or fatty acid of the present disclosure can be performed in any environment described herein in which the microorganism or composition of the present disclosure can be applied. The method of decomposing and removing oil and fat and/or fatty acid of the present disclosure can use any additional component described herein, which can be used in combination with the microorganism or composition of the present disclosure.

In one embodiment, the method of decomposing and removing oil and fat and/or fatty acid of the present disclosure comprises a step of adding the microorganism or composition of the present disclosure to an oil and fat decomposition tank, wherein the addition can be continuous or successive. The HRT (hydraulic retention time) in an oil and fat decomposition tank is generally 12 hours or longer, preferably 18 hours or longer, more preferably 20 hours or longer, and still more preferably 24 hours or longer. If 80% or more reduction in the n-Hex value is expected in wastewater with n-Hex exceeding 10000 mg/L, the HRT can generally be 18 hours or longer, preferably 20 hours or longer, and more preferably 24 hours or longer. If 80% or more reduction in the n-Hex value is expected in wastewater with an n-Hex value of 3000 mg/L or less, the HRT can generally be 8 hours or longer, preferably 12 hours or longer, and more preferably 18 hours or longer.

The microorganism concentration in an oil and fat decomposition tank can be dependent on the oil and fat and/or fatty acid concentration in wastewater. The bacterial concentration can be maintained higher at a higher oil and fat and/or fatty acid concentration. In case of foaming in the oil and fat decomposition tank, defoaming measures such as reducing the HRT, showering, or adding a defoamer may be taken. However, defoamers can inhibit the growth of microorganisms, so it is desirable to set the amount to be added while taking this into consideration.

For low concentration wastewater with an n-Hex value of water flowing in of about 300 mg/L or less, the n-Hex value of water flowing out from an oil and fat decomposition tank is preferably 60 mg/L or less, and more preferably 30 mg/L or less. For moderate concentration wastewater with an n-Hex value of water flowing in of about 3000 mg/L, the n-Hex value of water flowing out from an oil and fat decomposition tank is preferably 600 mg/L or less, more preferably 300 mg/L or less, still more preferably 150 mg/L or less, and most preferably 30 mg/L or less. For high concentration wastewater with an n-Hex value of water flowing in of about 10000 mg/L, the n-Hex value of water flowing out from an oil and fat decomposition tank is preferably 1000 mg/L or less, more preferably 500 mg/L or less, still more preferably 100 mg/L or less, and most preferably 30 mg/L or less. For high concentration wastewater with an n-Hex value of water flowing in of about 30000 mg/L or greater, the n-Hex value of water flowing out from an oil and fat decomposition tank is preferably 3000 mg/L or less, more preferably 1000 mg/L or less, and still more preferably 300 mg/L or less.

In one embodiment, the n-Hex value of oil and fat and/or fatty acid-containing wastewater can be reduced by preferably 80% or greater, more preferably 90% or greater, still more preferably 95% or greater, and most preferably 99% or greater by the method of the present disclosure. As a result, the n-Hex value of water flowing out from an oil and fat decomposition tank can be reduced in most wastewater to less than 30 mg/L, which is the reference value of release into the sewage system at many municipalities. If such a reference value is achieved, even the main treatment such as active sludge treatment in the later stage would not be needed in terms of just the n-Hex value.

The amount of microorganism added does not need to be increased in water flowing out from an oil and fat decomposition tank. The amount of microorganisms added in water flowing out is preferably 0.01-fold or greater, more preferably 0.1-fold or greater, still more preferably 0.5-fold or greater, and most preferably 1-fold or greater with respect to the amount added.

The method of decomposing and removing oil and fat and/or fatty acid of the present disclosure can comprise an additional step besides the steps described above. Examples of such a step include a step of returning a part or all of the water flowing out from an oil and fat decomposition tank again to the oil and fat decomposition tank and the like. However, the method of the present disclosure attains a sufficient effect of decomposing oil and fat and/or fatty acid without such a returning process, so that it is not essential to return a part or all of the water flowing out from an oil and fat decomposition tank again to the oil and fat decomposition tank.

### (General technology)

The molecular biological methodology, biochemical methodology, and microbiological methodology used herein are well-known and conventionally used in the art, which are described, for example, in Savli, H., Karadenizli, A., Kolayli, F., Gundes, S., Ozbek, U., Vahaboglu, H. 2003. Expression stability of six housekeeping genes: A proposal for resistance gene quantification studies of Pseudomonas aeruginosa by real-time quantitative RT-PCR. J. Med. Microbiol. 52: 403-408., Marie-Ange Teste, Manon Duquenne, Jean M Francois and Jean-Luc Parrou 2009. Validation of reference genes for quantitative expression analysis by real-time RT-PCR in Saccharomyces cerevisiae. BMC Molecular Biology 10: 99, Seiji Ishii, Hiroshi Okumura, Chiyo Matsubara, Fumi Ninomiya, Hiroshi Yoshioka, 2004, "Netsukannousei Polima wo Mochiita Suichuyubun no Kani Sokutei Hoho [Simple method of measuring oil-in-water content using heat sensitive polymer]", Vol 46, No.12, "Journal of water and waste", or the like. Relevant portions thereof (which may be the entire document) are incorporated herein by reference.

### (Note)

As used herein, "or" is used when "at least one or more" of the listed matters in the sentence can be employed. When explicitly described herein as "within the range" of "two values", the range also includes the two values themselves.

Reference literatures such as scientific literatures, patents, and patent applications cited herein are incorporated herein by reference to the same extent that the entirety of each document is specifically described.

As described above, the present disclosure has been described while showing preferred embodiments to facilitate understanding. The present disclosure is described hereinafter based on Examples. The above descriptions and the following Examples are not provided to limit the present disclosure, but for the sole purpose of exemplification. Thus, the scope of the present disclosure is not limited to the embodiments and Examples specifically described herein and is limited only by the scope of claims.

### [Examples]

The Examples are described hereinafter. When required, organisms used in the following Examples were handled in compliance with the guidelines stipulated by the Nagoya University, regulatory agency, or the Cartagena Protocol. For reagents, the specific products described in the Examples were used. However, the reagents can be substituted with an equivalent product from another manufacturer (Sigma-Aldrich, Fuji Film, Wako Pure Chemical, Nacalai Tesque, R & D Systems, USCN Life Science INC, Kanto Chemical, Funakoshi, Tokyo Chemical Industry, Merck, or the like).

### (Example 1: Identification of microorganism that can assimilate/decompose trans fatty acid-containing oil and fat)

A sample was collected from a river near a food factory from which oil and fat-containing wastewater flows out, and microorganism were isolated therefrom. To study whether the isolated microorganisms can be cultured in a low temperature environment (15°C), an agar medium comprising canola oil as the sole carbon source was streaked with each isolated microorganism for inoculation and cultured for 5 days at 15°C. As a result, microorganisms that can be cultured at a low temperature and use oil and fat as a nutrient source were found.

The ability to decompose oil and fat at a low temperature (15°C) of each microorganisms was studied. A colony of each microorganism was inoculated using a toothpick into a 20 mL inorganic salt medium comprising 10 g/L of canola oil as the sole carbon source, and was cultured in a 100 mL Erlenmeyer flask. As a result, microorganisms that decompose and assimilate oil and fat and grow thereon at a low temperature were found.

The ability to decompose/assimilate trans fatty acid of each microorganism was then studied. Each microorganism was inoculated into 2 mL of inorganic salt medium to which elaidic acid was added at a final concentration of 0.2% as the sole carbon source, so that the final concentration would be OD₆₆₀ = 0.04. The microorganisms were cultured for 24 hours at 28°C and at 130 rpm in a 15 mL Harmony centrifuge tube (LMS, Tokyo Japan). As a result, microorganisms that can decompose and assimilate trans fatty acid, i.e., elaidic acid, and grow were found.

These tests found that a certain isolated microorganism strain was capable of being cultured in a low temperature environment with oil and fat as a nutrient source, and assimilating (decomposing) oil and fat at a low temperature, and decomposes/assimilates trans fatty acid. This strain was named KH-1.

To further characterize KH-1, the genetic sequence of 16S rDNA was analyzed. As a result, KH-1 was identified as Burkholderia arboris. Comparison of the whole genome sequencing with a bacterial strain of the same species that were previously isolated using RAST (Rapid Annotation using Subsystem Technology, http://rast.nmpdr.org) found that there is a difference in genome size and found mutations involving deletion/insertion at several dozen sites. Further, variants were detected at several hundreds of sites from SNP/INDEL analysis using CLC Genomics server 9.0. The above results revealed that KH-1 was a novel strain of Burkholderia arboris.

Here, analysis was performed with the focus on KH-1, but the ability to decompose oil and fat and/or fatty acid of other stains can also be identified by studying the ability to decompose oil and fat and/or fatty acid by the same test.

The Burkholderia arboris KH-1 strain was streaked on an agar medium comprising canola oil (Nissin canola oil, Nissin Oilio, Tokyo, Japan) as the sole carbon source, and cultured for 5 days at 15°C. The results thereof are shown in Figure **1****.**

Since the KH-1 strain grew well, the KH-1 strain was recognized to be alive in an oil and fat-containing environment.

### (Example 2: Comparison of abilities to decompose oil and fat containing trans fatty acid with other microorganisms)

Trans fatty acid decomposition activity The activity of KH-1 to decompose trans fatty acid, elaidic acid (trans form of oleic acid) was compared with that of BioRemove 3200 (BR3200) (Novozymes, Denmark) (Figure **2**).

A stock solution was prepared by dissolving 2% elaidic acid in a 2.5% Triton X-100 (Sigma-Aldrich) solution. Since elaidic acid is a solid at normal temperature, 4 ml of the stock solution was heated at 65°C and added to 40 ml of inorganic salt medium (Na₂HPO₄ at 3.5 g/L, KH₂PO₄ at 2.0 g/L, (NH₄)₂SO₄ at 4.0 g/L, MgCl₂ . 6H₂O at 0.34 g/L, FeSO₄·7H₂O at 2.8 mg/L, MnSO₄·5H₂O at 2.4 mg/L, CoCl₂·6H₂O at 2.4 mg/L, CaCl₂·2H₂O at 1.7 mg/L, CuCl₂·2H₂O at 0.2 mg/L, ZnSO₄·7H₂O at 0.3 mg/L, and NaMoO₄ at 0.25 mg/L), and then subjected to an autoclave. The final concentrations of Triton X-100 and elaidic acid were 0.25% and 0.2%, respectively. KH-1 cultured overnight in an LB medium was washed twice with a PBS medium and then concentrated using an MX-100 high speed microcentrifuge (Tomy Seiko, Tokyo, Japan), and inoculated into the medium described above, so that the final concentration would be OD₆₆₀ = 0.04 (HITACHI U-2810 spectrophotometer (Hitachi, Tokyo, Japan)). BR3200 was suspended in 100 ml of distilled water after measuring out 1 g with a base, and then stirred for 30 minutes and inoculated so that the initial inoculation concentration would be the same as that of KH-1 (the final concentrations of each bacteria were all adjusted to 4 x 10⁵ cells/ml). The bacteria were cultured for 24 hours at 28°C and at 130 rpm. Samples were analyzed using TLC (Figure **2A**) and oil content measuring reagent kit (Kyoritsu Chemical Check Lab, Tokyo, Japan) (measuring reagent kit using a method of measuring poly(N-isopropylacrylamide) extracted substances) (Figure **2B**). It was found as a result that KH-1 almost completely decomposes/assimilates 0.2% elaidic acid in 24 hours (at least decreased to a concentration of 200 mg/L (0.02%) or less). Meanwhile, elaidic acid remained with BR3200.

### (Example 3: Comparison of abilities to decompose trans fatty acid-containing oil and fat)

Ability of microorganisms to decompose trans fatty acid-containing oil and fat

The KH-1 strain was inoculated into a TBS buffer (20 mM Tris, 150 mM NaCl, pH 7.0) to which trielaidin was added such that the final concentration would be 0.1%, so that the final concentration according to bacterial optical density would be OD₆₆₀ = 0.04 by using a HITACHI U-2810 spectrophotometer (Hitachi, Tokyo, Japan). This was cultured in a Falcon tube (130 rpm, 28°C), and samples were collected after 5 days. The samples were made visible by TLC (applied to a silica gel plate, developed with a chloroform:acetone:methanol (96:4:2) solution and then treated with molybdic acid n-hydrate (2.4 g in 60 ml of ethanol)) (Figure **3A**). Data for cases where only TBS buffer was added is also shown as a control.

It was confirmed from the results that KH-1 has the ability to decompose/assimilate trans fatty acid-containing oil and fat and decompose/assimilate trans fatty acid.

Ability of microorganism culture supernatant to decompose trans fatty acid-containing oil and fat

In the same manner, 1 mL of the KH-1 strain culture supernatant described above (estimated lipase concentration: 50 u/ml) or TBS buffer (pH of 7.0) and 0.05 mL of trielaidin stock solution (2% trielaidin and aqueous 5% Triton X-100 solution) were added to a 1.5 mL microtube (Watson, Tokyo, Japan) to prepare a reaction solution so that the final concentration of trielaidin would be 0.1%. This was incubated for 24 hours at 130 rpm and at 28°C to obtain samples. The samples were extracted with an equal volume of chloroform, and 5 µl was subjected to TLC. The samples were made visible by TLC (applied to a silica gel plate, developed with a chloroform:acetone:methanol (96:4:2) solution, and then treated with molybdic acid n-hydrate (2.4 g in 60 ml of ethanol)) (Figure **3B**).

It was confirmed from the results that trans fatty acid-containing oil and fat (trielaidin) can be decomposed into trans fatty acid (elaidic acid) even if the supernatant of KH-1 is used.

Actual wastewater was used to compare the abilities of KH-1 and BR3200 to decompose oil and fat (Figure **4**).

Wastewater samples containing a large amount of trans fatty acid-containing oil and fat from a food factory using hydrogenated oil and fat were supplemented with phosphorus and nitrogen content(ammonium sulfate) corresponding to an inorganic salt medium for culture. KH-1 was cultured in an LB medium and washed twice with PBS medium and then inoculated at 1 × 10⁶ cells/ml. BR3200 was inoculated at 1 × 10⁸ cells/ml, which is a concentration that is 10-fold of the manufacturer recommended concentration. The bacteria were cultured at 28°C, and samples were collected after 24 hours and 48 hours. The samples were analyzed using TLC (applied to a silica gel plate, developed with a chloroform:acetone:methanol (96:4:1) solution and then made visible with molybdic acid n-hydrate) (Figure **4A**) and oil content measuring reagent kit (corresponding to normal hexane extraction, described above) (Figure **4B**). The progress of decomposition was slow for BR3200 even at an excessive amount, whereas KH-1 exhibited an excellent ability to decompose.

### (Example 4: Ability of the strain KH-1 to decompose oil and fat at 15°C)

Burkholderia arboris KH-1 was inoculated into 3L of inorganic salt medium (composition described above) comprising 10 g/L of canola oil (Nissin canola oil, Nissin Oilio), so that the final concentration according to the bacterial optical density would be OD₆₆₀ = 0.03 by using a HITACHI U-2810 spectrophotometer (Hitachi, Tokyo, Japan). This was cultured in a 5L volume fermenter (250 rpm, under 200 ml/min air circulation, pH of 7.0, and 15°C), and samples were collected over time. Bacteria were removed from the sampled culture by centrifugation. Oil content corresponding to a normal hexane value in the supernatant was measured with an oil content measuring reagent kit (normal hexane extraction described above) (Figure **5A**).

The total fatty acid (total amount of fatty acid in triglyceride and free fatty acid) was quantified by gas chromatography (Figure **5B**) **.** Specifically, 3 ml of culture supernatant was acidified with hydrochloric acid, and double the amount of ethyl acetate was added. After 5 minutes of agitating and then centrifugation, 1 ml of the ethyl acetate layer was transferred to an organic solvent resistant tube and completely evaporated. 4 ml of methanolysis solution prepared by mixing methanol:sulfuric acid = 17:3 was added. The mixture was heated for 2 hours at 100°C to convert all fatty acids into their methyl esters. A solution prepared by mixing chloroform:pure water = 1:1 was added and thoroughly agitated, then a chloroform layer and 0.5% methyl octanoate (internal standard) were mixed at 1:1. The mixture was analyzed by gas chromatography with an FID detector (GC-17A (Shimadzu, Kyoto, Japan)).

It was confirmed from these results that KH-1 has a high ability to decompose/assimilate oil and fat even at a low temperature.

### (Example 4A: Ability of the strain KH-1 to assimilate trans fatty acid and trans fatty acid-containing oil and fat at 15°C)

The ability of the KH-1 strain to assimilate elaidic acid or trielaidin was evaluated by culturing a KH-1 strain in a medium containing them as the sole carbon source.

A KH-1 strain was inoculated into 2 mL of an inorganic salt medium (composition described above, pH of 7) supplemented with a stock solution (aqueous 2.5% Triton X-100 solution) of elaidic acid or trielaidin such that the final concentration of elaidic acid or trielaidin would be 0.1%, so that the final concentration according to bacterial optical density would be OD₆₆₀ = 0.08 by using HITACHI U-2810 spectrophotometer (Hitachi, Tokyo, Japan).

This was cultured for 5 days at 15°C, and the growth of microorganisms was observed. Figure **5C** shows results for each of elaidic acid and trielaidin-added media. Results from not adding a microorganism is also shown as a control.

The medium was observed to be cloudy in a KH-1 strain-inoculated sample. This demonstrated that the strain KH-1 can grow under an environment comprising elaidic acid or trielaidin as the sole carbon source at 15°C and has the ability to assimilate these compounds.

### (Example 5: Ability of the strain KH-1 to decompose oil and fat at 28°C)

In the same manner as the descriptions above, the ability of KH-1 to decompose oil and fat in 28°C culture was also evaluated (Figure **6**).

Residual oil content in the culture was analyzed by thin-layer chromatography (TLC) (Figure **6A**). In detail, to 3 ml of sample supernatant, an equal amount of ethyl acetate was added, and the mixture was agitated for 5 minutes. After recovering the ethyl acetate layer and completely evaporating the solvent, the resultant was dissolved in 300 µL of chloroform. 3 µl of the resulting mixture was applied to a silica gel plate and developed with a chloroform:acetone (96:4) solution. After the development, a 4% (w/v) 12 Molybdo(IV) phosphoric acid ethanol solution was sprayed to the plate, and the plate was heated for 12 minutes at 110°C to make oil and fat and free fatty acid visible. The amounts of oil and fat and the fatty acid, which is the decomposition product thereof, remaining in the medium were compared. As a result of the decomposition of triglyceride with lipase secreted by the microorganisms, free fatty acid that is a hydrolysate was detected (24h). Such fatty acid was also decomposed with the passage of time. KH-1 completely eliminated free fatty acid in 48h.

In the same manner as Example 4, the total fatty acid (total amount of fatty acid in triglyceride and free fatty acid) in the examination at 28°C was quantified by gas chromatography (Figure **6B**). In the same manner as Example 4, residual oil and fat in the examination at 28°C was also measured with an oil content measuring reagent kit (Figure **6C**) .

KH-1 also retained an excellent ability to decompose oil and fat at 28°C.

### (Example 6: Ability to decompose various trans fatty acids)

The activity of KH-1 to decompose trans fatty acids palmitelaidic acid (16:1) and vaccenic acid (18:1) was compared with that of BioRemove 3200 (BR3200) (Novozymes, Denmark).

KH-1 and BR3200 were inoculated into 5 mL of inorganic salt medium (composition described above, pH of 7) prepared such that the final concentration of palmitelaidic acid or vaccenic acid would be 0.2% and the final concentration of Triton X100 would be 0.25%, so that the final concentration of KH-1 according to bacterial optical density would be OD₆₆₀ = 0.05, and the final concentration of BR3200 would be about 5 × 10⁶ CFU/g that corresponds to OD₆₆₀ = 0.05 using a HITACHI U-2810 spectrophotometer (Hitachi, Tokyo, Japan). This was cultured with shaking at 130 rpm for 48 hours or 72 hours at 15°C or for 24 hours at 28°C, and then culture supernatant of each strain was obtained. A control sample that did not use a microorganism was also prepared.

Subsequently, residual oil content in culture was analyzed by thin-layer chromatography (TLC). Specifically, fatty acids were extracted with chloroform at an amount that is 1/2 of the sample. 6 µl of the extract was applied to a silica gel plate and developed with a chloroform:acetone:methanol (96:4:2) solution. After the development, fatty acids were made visible with a 12 Molybdo(IV) phosphoric acid ethanol solution in the same manner as Example 5. The amounts of fatty acids remaining in the medium were compared (Figures **8** and **9**).

KH-1 was able to completely decompose palmitelaidic acid and vaccenic acid within 24 hours at 28°C, and within 72 hours at 15°C. On the other hand, BR3200 did not have the ability to decompose these fatty acids. In this manner, the microorganism of the present disclosure can be capable of decomposing various trans fatty acids.

### (Example 7: Comparison of KH-1 strain with detergents)

Ventilator filters with oil stains were washed by leaving the filters soaked at room temperature (25°C) in a culture supernatant of KH-1 (culture conditions: KH-1 was inoculated into the inorganic salt medium (composition described above) supplemented with 1% canola oil, so that initial concentration would be OD₆₆₀ = 0.01, and cultured at 28°C for 24 hours; supernatant obtained by centrifuging after the culture to remove bacteria was used), a detergent for oil (natural enzyme detergent Nicoeco for kitchen (Nicoeco, Nagano, Japan), diluted with water 143-fold in accordance with the instruction manual), and a multipurpose detergent (Family® (Kao, Tokyo, Japan) diluted 666-fold in accordance with the instruction manual) (Figure **7**). The multipurpose detergent and the detergent for oil could not completely remove oil stains after 4 hours of soak washing, but with KH-1, the filter became pure white like a new product with 1 hour of soak washing.

### (Example 8: Acquisition of other strains)

A sample was collected from a river near a food factory from which oil and fat-containing wastewater was discharged, and microorganism were isolated therefrom. The inventors studied whether the isolated microorganisms could decompose trans fatty acid or trans fatty acid-containing oil and fat at a low temperature (15°C). As a result, microorganisms that could decompose trans fatty acid and trans fatty acid-containing oil and fat at a low temperature were found. These microorganism strains were named KH-1AL1, KH-1AL2, and KH-1AL3, respectively.

To further characterize KH-1AL1, KH-1AL2, and KH-1AL3, the genetic sequence of 16S rDNA was analyzed.
KH-1AL1 was identified as Burkholderia ambifaria because the partial base sequence of 16 rDNA matched Burkholderia ambifaria with 100% homology.
KH-1AL2 was classified to the same group as B. seminalis, B. territorii, and B. cepacia (homology in the partial base sequence of 16 rDNA was 99.7%, 99.7%, and 99.8%, respectively) on the phylogenetic tree, with the partial base sequence of 16 rDNA matching Burkholderia contaminans with 99.9% homology. As a result, KH-1AL2 was identified as a bacterium belonging to the Burkholderia cepacia complex.
KH-1AL3 was classified to the same group as B. seminalis, B. territorii, and B. cepacia (homology in the partial base sequence of 16 rDNA was 99.7%, 99.7%, and 99.8%, respectively) on the phylogenetic tree, with the partial base sequence of 16 rDNA matching Burkholderia contaminans with 99.9% homology. As a result, KH-1AL3 was identified as bacteria of the Burkholderia cepacia complex.

Burkholderia cepacia complex is a classification of microorganisms of the genus Burkolderia that are genetically very close, including ambifaria, anthina, arboris, cenocepacia, cepacia, contaminans, diffusa, dolosa, lata, latens, metallica, multivorans, pseudomultivorans, puraquae, pyrrocinia, seminalis, stabilis, stagnalis, territorii, ubonensis, and vietnamiensis (Martina P et al., Int J Syst Evol Microbiol. 2018 Jan; 68(1):14-20.).

As a result of analysis, various bacteria belonging to the Burkholderia cepacia complex exhibited a high ability to decompose oil and fat and/or fatty acid, so that bacteria belonging to the Burkholderia cepacia complex are expected to be particularly useful.

### (Example 9: Ability of three additional strains to decompose oil and fat at 15°C)

KH-1AL1, KH-1AL2, and KH-1AL3 were each pre-cultured in an LB medium and then washed twice in PBS with centrifugation for 10 minutes at 25°C and at 3000 g to prepare samples of each bacterial strain.

These three bacterial strain samples were inoculated into an inorganic salt medium (composition described above) with a pH of 7, comprising 1% canola oil (Nissin canola oil, Nissin Oilio), so that the final concentration according to bacterial optical density would be OD₆₆₀ = 0.02 by using a HITACHI U-2810 spectrophotometer (Hitachi, Tokyo, Japan). This was cultured for 96 hours at 15°C, and samples were collected over time. Bacteria were removed from the sampled culture by centrifugation. Each of the supernatants of the three bacterial strains, in accordance with the same procedures as the Examples described above, was subjected to thin-layer chromatography (TLC) to analyze residual oil content in culture, gas chromatography to quantify total fatty acid (total amount of fatty acid in triglyceride and free fatty acid), and measurement to determine oil content corresponding to a normal hexane value with an oil content measuring reagent kit.

The results are shown in Figures **10** to **12****.** It was confirmed from these results that KH-1AL1, KH-1AL2, and KH-1AL3 can efficiently decompose/assimilate oil and fat at a low temperature.

### (Example 10A: Comparison of abilities to decompose trans fatty acid-containing oil and fat at 28°C by culture supernatant among strains)

KH-1, KH-1AL1, KH-1AL2, and KH-1AL3 were each inoculated into an inorganic salt medium (composition described above, pH of 7) containing trielaidin of the final concentration of 0.2%, so that the final concentration according to bacterial optical density would be OD₆₆₀ = 0.08 using a HITACHI U-2810 spectrophotometer (Hitachi, Tokyo, Japan). This was cultured for 6 days at 28°C and then culture supernatant of each strain was obtained. A control sample that did not use a microorganism was also prepared.

After filtering the culture supernatant obtained from each strain using a cellulose acetate membrane filter with a pore size of 0.45 µm (ADVANTEC, Tokyo, Japan), trielaidin and Triton X100 were added such that the final concentrations would be 0.4% and 0.5%, respectively. The culture supernatants were incubated for 24 hours at 37°C while shaking at 130 rpm.

Subsequently, residual oil content in the culture was analyzed by thin-layer chromatography (TLC). Specifically, oil and fat and free fatty acids were extracted with chloroform at an amount that is 1/2 of the sample. 5 µl of the extract was applied to a silica gel plate and developed with a chloroform:acetone:methanol (96:4:2) solution. After the development, oil and fat and free fatty acid were made visible with a 12 Molybdo(IV) phosphoric acid ethanol solution in the same manner as Example 5, and the amounts of oil and fat and fatty acids, which are the decomposition product thereof, remaining in the medium were compared (Figure **13**).

As a result, the culture supernatants of KH-1, KH-1AL1, KH-1AL2, and KH-1AL3 were confirmed to be able to efficiently decompose trans fatty acid-containing oil and fat. This result can be understood to be due to the secretion of lipase by these strains, and suggest that these microorganism by themselves or the culture supernatant thereof can be useful in the decomposition of trans fatty acid-containing oil and fat.

### (Example 10B: Comparison of abilities to decompose/assimilate trans fatty acid at 28°C among strains)

KH-1, KH-1AL1, KH-1AL2, and KH-1AL3 were each inoculated into an inorganic salt medium (composition described above, pH of 7) containing elaidic acid of the final concentration of 0.2% and Triton X100 of the final concentration of 0.25%, so that the final concentration according to bacterial optical density would be OD₆₆₀ = 0.04 using a HITACHI U-2810 spectrophotometer (Hitachi, Tokyo, Japan). This was cultured for 20 hours at 28°C while shaking at 130 rpm, and then culture supernatant of each strain was obtained. A control sample that did not use a microorganism was also prepared.

Subsequently, residual oil content in the culture was analyzed by thin-layer chromatography (TLC). Specifically, fatty acids were extracted with chloroform at an amount that is 1/2 of the sample. 5 µl of the extract was applied to a silica gel plate and developed with a chloroform:acetone:methanol (96:4:2) solution. After the development, free fatty acids were made visible with a 12 Molybdo(IV) phosphoric acid ethanol solution in the same manner as Example 5, and the amounts of fatty acids remaining in the medium were compared (Figure **14** left). Further, the residual oil content corresponding to a normal hexane value was measured with an oil content measuring reagent kit by the same procedure as Example 4 (Figure **14** right) .

As a result, KH-1, KH-1AL1, KH-1AL2, and KH-1AL3 were confirmed to be able to efficiently decompose trans fatty acid. This result suggests that each of these strains can be useful in removing oil comprising trans fatty acid. Together with the results in Example 10A, these microorganisms are expected to be able to completely decompose trans fatty acid-containing oil and fat (e.g., trielaidin).

### (Example 10C: Comparison of abilities to decompose/assimilate trans fatty acid at 15°C among strains)

KH-1, KH-1AL1, KH-1AL2, and KH-1AL3 were each inoculated into an inorganic salt medium (composition described above, pH of 7) containing elaidic acid of the final concentration of 0.2% and Triton X100 of the final concentration of 0.25%, so that the final concentration according to bacterial optical density would be OD₆₆₀ = 0.08 using a HITACHI U-2810 spectrophotometer (Hitachi, Tokyo, Japan). This was cultured for 72 hours at 15°C while shaking at 130 rpm, and then culture supernatant of each strain was obtained. A control sample that did not use a microorganism was also prepared.

Subsequently, residual oil content in the culture was analyzed by thin-layer chromatography (TLC). Specifically, free fatty acid was extracted with chloroform at an amount that is 1/2 of the sample. 5 µl of the extract was applied to a silica gel plate and developed with a chloroform:acetone:methanol (96:4:2) solution. After the development, free fatty acid was made visible with a 12 Molybdo(IV) phosphoric acid ethanol solution in the same manner as Example 5, and the amounts of fatty acids remaining in the medium were compared (Figure **15** left). Further, the residual oil content corresponding to a normal hexane value was measured with an oil content measuring reagent kit by the same procedure as Example 4 (Figure **15** right) .

As a result, KH-1, KH-1AL1, KH-1AL2, and KH-1AL3 were confirmed to be able to efficiently decompose trans fatty acid. This result suggests that each of these strains can be useful in removing oil comprising trans fatty acid. Together with the results in Example 10A, these microorganisms are expected to be able to completely decompose trans fatty acid-containing oil and fat (e.g., trielaidin).

### (Example 11: Application to food waste treatment)

KH-1 was inoculated into an extinguishing type of disposer so that the concentration would be 1.2 × 10⁷ cells/mL, and food waste was treated for 24 hours at 30°C. The normal hexane value in wastewater discharged from the food waste disposer was measured. A reduction in the normal hexane value (2.22 g/L), which is significantly decreased from a control (3.26 g/L) with no addition of KH-1, was found. In this regard, food waste in the extinguishing type of disposer was a food waste produced from a kitchen in a restaurant wherein bones and shells were removed. The food waste processing capability of the extinguishing type of disposer was 20 kg/day, and the amount of water supply and discharge was 38.4 L/day.

### (Example 12: Use within an apparatus)

KH-1 is cultured to 2 × 10¹⁰ cells/mL in an inorganic salt medium comprising 10 mL/L of canola oil to prepare the undiluted culture solution. This is diluted 10-fold to prepare a microbial formulation (2 × 10⁹ cells/mL). This is refrigerated in a microorganism storage tank of an automatic amplifying and adding apparatus as seed bacteria. The seed bacteria are automatically inoculated daily at an amount of 1/100 into an inorganic salt medium in a culture amplification tank of the apparatus, and cultured until the number of microorganism became 100-fold, i.e., same cell concentration as the microbial formulation. This is administered at 1/1000 of the amount of wastewater in an oil decomposition and treatment tank so that the concentration of decomposing bacteria in oil treating water would be 2 × 10⁶ cells/mL, and wastewater from a food factory discharging wastewater containing a large amount of trans fatty acid-containing oil and fat is decomposed and treated for 24 hours. The season at the time is winter. The water temperature during treatment varies between 12 to 17°C.

As a result, a significant reduction in the normal hexane value is observed compared to a control without addition of a microorganism.

### (Example 13: Other embodiments)

Carriers such as charcoal, various plastics, and ceramic fragments are added to a grease trap, and a suitable amount (e.g., 1 × 10⁶ cells/mL) of KH-1 is automatically added every day after the end of business day at a restaurant. Water is collected before the start of business every day to analyze the normal hexane value. After one week, a significant decrease in the normal hexane value is observed compared to a control without addition of KH-1, and an effect is observed on the appearance of the grease trap itself such as reduced adhesion or floating of oil.

### (Note)

As described above, the present disclosure is exemplified by the use of its preferred embodiments. However, it is understood that the scope of the present disclosure should be interpreted solely based on the Claims. It is also understood that any patent, any patent application, and any references cited herein should be incorporated herein by reference in the same manner as the contents are specifically described herein.

### [Industrial Applicability]

The present disclosure provides a microorganism having an ability to decompose oil and fat and a composition comprising the same. Burden on the environment due to wastewater from food factories containing a large amount of oil and fat and the like can be reduced by using such a microorganism or composition.

### [Reference to Deposited Biological Material]

KH-1 strain (NITE BP-02731)
KH-1AL1 strain (NITE ABP-02977)
KH-1AL2 strain (NITE ABP-02978)
KH-1AL3 strain (NITE ABP-02979)

## Claims

1. A bacterium in the family Burkholderiaceae, having an ability to assimilate trans fatty acid-containing oil and fat.

2. A bacterium in the family Burkholderiaceae, having an ability to assimilate trans fatty acid.

3. A bacterium in the family Burkholderiaceae, having an ability to decompose trans fatty acid.

4. A bacterium in the family Burkholderiaceae, having an ability to decompose trans fatty acid-containing oil and fat.

5. A bacterium in the family Burkholderiaceae, having an ability to decompose oil and fat at 15°C.

6. The bacterium in the family Burkholderiaceae of any one of claims 1 to 4, wherein the ability to assimilate or decompose is retained at 15°C.

7. The bacterium of any one of claims 1 to 6, which is a bacterium of the genus Burkholderia.

8. The bacterium of any one of claims 1 to 7, which is a microorganism belonging to Burkholderia arboris, Burkholderia ambifaria, or Burkholderia cepacia complex.

9. The bacterium of any one of claims 1 to 8, which is a KH-1 strain (bacterial strain identified by accession number NITE BP-02731), a KH-1AL1 strain (bacterial strain identified by receipt number NITE ABP-02977), a KH-1AL2 strain (bacterial strain identified by receipt number NITE ABP-02978), or a KH-1AL3 strain (bacterial strain identified by receipt number NITE ABP-02979) of a bacterium in the genus Burkholderia, or a derivative strain thereof that has a feature of the bacterium of any one or more of claims 1 to 7.

10. An oil decomposing agent comprising the bacterium of any one of claims 1 to 9.

11. The oil decomposing agent of claim 10, further comprising an oil treating component.

12. A kit for decomposing oil, comprising the bacterium of any one of claims 1 to 9 or the oil decomposing agent of claim 10, and an additional oil treating component.

13. A method of decomposing and removing oil, comprising causing the bacterium of any one of claims 1 to 9 or the oil decomposing agent of claim 10 or 11 to act on a subject of treatment.

14. The method of claim 13, wherein the subject of treatment comprises trans fatty acid or trans fatty acid-containing oil and fat.
